# EUROPÄISCHE PATENTSCHRIFT

(11) **EP 0 738 282 B1**
(45) Veröffentlichungstag und Bekanntmachung des Hinweises auf die Patenterteilung: **24.03.1999**
(21) Anmeldenummer: 95906286.0
(22) Anmeldetag: 03.01.1995
(51) Int. Cl.: C07K 16/46, G01N 33/577

(54) **NEUE CHIMÄRE ANTIKÖRPER, DEREN VERWENDUNG UND VERFAHREN ZU IHRER HERSTELLUNG**
NOVEL CHIMERIC ANTIBODIES, THEIR USE AND PROCESS FOR PRODUCING THEM
NOUVEAUX ANTICORPS CHIMERES, LEUR UTILISATION ET LEUR PROCEDE DE PRODUCTION

(30) Priorität: 08.01.1994 DE 4400352
(43) Veröffentlichungstag der Anmeldung: 23.10.1996
(73) Patentinhaber: Batsford, Stephen, 79418 Schliengen (DE)
(72) Erfinder: Batsford, Stephen, 79418 Schliengen (DE)
(74) Vertreter: De Clercq, Ann G. Y.
(86) Internationale Anmeldenummer: EP9500005
(87) Internationale Veröffentlichungsnummer: WO9518831

(56) Entgegenhaltungen:
- CA-A- 1 329 545
- THE JOURNAL OF IMMUNOLOGY, Bd. 139,Nr. 7, 1.Oktober 1987 BALTIMORE, MD, VSA, Seiten 2367-2375, M. GLENNIE ET AL. 'Preparation and performance of bispecific F(ab'gamma)2 antibody containing tioether-linked Fab'gamma fragments.'
- THE JOURNAL OF EXPERIMENTAL MEDICINE, Bd. 174,Nr. 6, 1.Dezember 1991 NEW YORK, NY, VSA, Seiten 1483-1489, K. PEPPEL ET AL. 'A tumor necrosis factor (TNF) receptor-IgG heavy chain chimeric protein as a bivalent antagonist of TNF activity.'

## Beschreibung

### Technisches Gebiet

Die vorliegende Erfindung bezieht sich auf das Gebiet der Immunologie und betrifft chimäre Antikörper und ihre Verwendung bei der Diagnostik. Inbesondere betrifft die vorliegende Erfindung chimäre Antikörper, welche aus einem ersten Antikörper bzw. Immunglobulin und einem zweiten Antikörper bzw. Immunglobulin zusammengesetzt sind, wobei die besagten Antikörper bzw. Immunglobuline von einander verschiedenen Ursprungs sind. Derartige cnimäre Antikörper können als diagnostische Mittel in einem sehr breiten Bereich auf dem serologisch-immunologischen Gebiet eingesetzt werden. Die besagten chimären Antikörper *per se* und ihre Bereitstellung, Verfahren zu ihrer Herstellung und ihre Verwendung in immundiagnostischen Verfahren und Assays werden beschrieben.

### Stand der Technik

Aus dem Stand der Technik sind verschiedene, aus Teilen unterschiedlicher Herkunft zusammengesetzte, chimäre oder über Linkermoleküle heterokonjugierte chimäre Antikörper bekannt. Ferner sind immunologische Verfahren bekannt, um Antikörper zu bestimmen

In der Arbeit von MORRISON, S. L. et al., Proc. Natl. Acad. Sci. USA 81, 6851 - 6855, 1984, werden chimäre Maus-Human Antikörper beschneben, bei denen Gene der variablen Region einer Maus mit Genen der konstanten Region vom menschlichen Immunglobulin verbunden wurden. Derartige, durch DNA Rekombination hergestellte chimäre Antikörper sollen klinische Anwendung finden und es wird vorgeschlagen, diese als immuntherapeutische und diagnostische Reagentien zu verwenden. Insbesondere sollen derartige humanisierte chimäre Antikörper das Auftreten von Immunogenitäten von in vivo verwendeter Antikörper zu verringern.

SHARON J. et al., Nature 309, 364 - 367, 1984, offenbaren die Expression eines chimären Genes, welches für die variable (V) Region der schweren Kette der Maus und die konstante (C) Region einer K leichten Kette (V_{H}C_{κ} ) kodiert. Das chimäre Gen konnte in einer Mäuse Myeloma Zellinie zu Expression gebracht werden

In der Publikation von SHIN U.-S., Biotherapy 3, 43 - 53, 1991, werden Strategien für die Herstellung von chimären Antikörpern beschrieben, wobei von genomischen, cDNA oder PCR amplifizierten Klonen ausgegangen wird. Die genetisch veränderten Immunglobuline werden nach Gentransfektion in Immunglobulin nicht-produzierenden Myelomazellen oder Transfektomas exprimiert.

Von MOCIKAT R. et al., Biotechniques 12(3), 355 - 356, 1992, wird ein PCR Verfahren offenbart für die Herstellung von chimären Antikörpern, welches die Klonierung von V - Gensegmenten umfaßt

In dem jüngst erschienenen Übersichtsartikel von LEON J. A. et al., Pharmac. Ther. 61, 237 - 278, 1994, werden monoklonalen Antikörpern eine hohe Bedeutung als wichtige Reagentien *inter alia* in der Diagnose eingeräumt. Hinsichtlich der in dieser Arbeit offenbarten chimären Antikörper werden ausschließlich solche beschrieben, die eine Kombination muriner VDJ Regionen der schweren Kette und VD Regionen der leichten Kette mit Humanen H und L konstanten Ketten umfassen. Oder solche, die mittels "complementarity determining regions (CDR) grafting" humanisiert worden sind, indem H und L hypervariable Regionen (CDR) der Maus mit konstanten Regionen des Menschen kombiniert wurden.

In der Publikation von BRENNAN, M. et al., Science 229, 81 - 83, 1985, wird die Herstellung bispezifischer Antikörper durch chemische Rekombination monoklonaler Immunglobulin G₁ Fragmente offenbart, wonach von zwei verschiedenen F(ab')₂ Fragmenten mit 2-Mercaptoethylamin in Gegenwart von Natriumarsenit und Ellman's Reagenz Fab'-TNB Derivate hergestellt und von diesen durch Reduktion Fab'-Thiole gebildet werden und anschließend die entsprechenden Hybrid-Dimeren durch Reoxidation gewonnen werden.

PEREZ, P. et al, Nature 316, 354 - 356, 1985, offenbaren über den Linker N-succinimidyl-3-(2-pyridyldithiol)propionat verbundene Antikörperheterokonjugate, wobei verschiedene OKT3 (anti-T3) enthaltende Antikörperheterokonjugate humanem Ursprungs verbunden wurden mit dem Effekt einer besseren Zielwirkung auf Zelloberflächenantigene, indem die T_{c} Zell Spezifitäten durch Behandlung mit derartigen Antikörperheteroaggregaten verändert werden.

Ähnliche Antikörperheteroaggregate werden von TITUS, J. A., J. Immunology, 138, 4018 - 4022, 1987, beschrieben. In diesem Fall wurden humane periphere Blut T Zellen gecoated mit einem Heteroaggregat, bestehend aus einen anti-T3(Fab) verbunden mit einem humanen antitumor monoklonalen Antikörper, wobei eine erhöhte Antitumorwirkung festgestellt wurde.

Die Verwendung von heterokonjugierten Antikörpern, CD3-spezifische oder FcR-spezifische monoklonale Antikörper kovalent verbunden mit Herpes Simplex Virus (HSV) spezifischen monoklonalen Antikörper zur Induktion von T Lymphozyten und NK Zellen zur spezifischen Lyse von HSV-infizierten Zellen wird von PAYA, C. V. et al., J. Immunol. 142, 666- 671, 1989, beschrieben.

CHARPIE, J. R. et al., Biochemistry 29, 6374 - 8378, 1990, beschreiben einen bispezifischen Antikörper bestehend aus einem monoklonalen Antikörper gegen einen Teil der β-Kette von humanem Fibrin kovalent über einen Linker verbunden mit einem monoklonalen Antikörper gegen die zweikettige Urokinase des Menschen.

Von GÖRÖG, G. et al., J. Immunol. Methods 123, 131 - 140, 1989, ist ein homogener Enzymimmunoassay bekannt, welcher chemisch hybridisierte Antikörperhälften nach der Methode von BRENNAN, 1985, loc. cit., verwendet.

Von MARTINEK, K. & TORCHILIN, V. P., Methods in Enzymology 137, 615 - 626, 1988, ist bekannt, daß intramolekulares Cross-Linking mittels bifunktioneller Reagentien die Thermostabilität von Enzymen erhöhen. Die Autoren spekulieren, daß ein solches Cross-Linking den ersten Schritt eines Thermoinaktivierungsprozesses, die Dissoziation oligomerer Enzyme in ihre Untereinheiten, verhindern könnte.

MAN, N. et al., J. Immunol Methods 125, 251 - 259, 1989, beschreiben die Herstellung von polykonalen und monoklonalen Antikörper durch Verwendung eines Glutaraldehyd-aggregierten Immunogens. Es wird unter anderem spekuliert, ob durch dieses Crosslinking eine stabilisierende Wirkung auf die native Konformation des betreffenden Immunogens hervorgerufen wird.

MILSTEIN, C. und CUELLO, A. C., Nature 305, 537 - 540, 1983, offenbaren Hybridimmunoglobuline bzw. bispezifische monoklonale Antikörper, welche durch Fusion von zwei Antikörper produzierenden Zellen (Hybrid-Hybridomas) erzeugt wurden.

In den letzten zehn Jahren ist eine Anzahl an Patentliteratur auf dem Gebiet chimärer Antikörper erschienen, die sich molekularbiologischer Methoden bedient, um über die Technik der DNA Rekombination und Produktion heterologer Proteine chimäre Antikörper mit veränderten Domänen bereitzustellen.

Aus der W086/01533 sind chimäre Antikörper bekannt, bei denen die konstanten Domänen eines nicht-humanen Antikörpers (beispielsweise von Maus oder von der Ratte) durch konstante Domänen des Menschen ersetzt worden sind.

In der W092/05274 werden Antikörper beschrieben, bei denen die CDRs (complementarity determining regions) der variablen Domäne der Antikörperkette von einer ersten Säugetierspezies stammen und der "framework"-Bereich der variablen und der konstanten Domäne von einem zweiten unterschiedlichen Säugetierspezies. Ein derartig veränderter Antikörper soll entweder die Struktur eines natürlichen Antikörper oder eines Fragments davon haben oder er kann auch chimär sein, indem er eine Antigenbindungsstelle und eine nicht-immunoglobulin Region enthält.

Der EP-A 0 125 023 ist die Herstellung eines chimären Immunglobulins zu entnehmen, dessen konstante Region aus einem ersten Säugetier stammt und dessen variable Region homolog zu einer zweiten, differenten Säugetierspecies ist.

Die Europäische Patentanmeldung EP-A 0 247 091 schlägt zur Herstellung genetisch veränderter chimärer Antikörper mit einer konstanten Region des Menschen und einer variablen nicht-Human Region vor, eine cDNA, die für die vollständige variable nicht-Human Domäne kodiert mit einem Vektor, der die genetische Information für die konstante Region enthält, zu verbinden.

Die EP-A 0 378 175 offenbart die Verwendung von chimären monoklonalen Antikörpern oder Fragmenten davon, die aus humanen Antikörpern bestehen, bei denen die variable Region ganz oder teilweise durch die entsprechenden Teile eines nicht-humanen monoklonalen Antikörpers der gewünschten Spezifität ersetzt sind. Derartige chimäre Antikörper sollen für diagnostische Tests zur quantitativen immunologischen Bestimmung von Substanzen im Blut oder Serum Verwendung finden. Dies soll insbesondere dadurch erfolgen, daß zwei gegen die zu untersuchende Substanz gerichtete monoklonale Antikörper R₁ und R₂ verwendet werden, wobei R₁ an eine feste Phase gebunden ist und R₂ eine Markierung trägt, und mindestens einer der besagten Antikörper ein chimärer Antikörper ist.

Von FOMSGAARD, A. & DINESEN B., J. Immunoassay 8, 333 - 350, 1987, wurde ein kombinierter, aus verschiedenen Schritten aufgebauter ELISA vorgeschlagen, um Antikörper nachzuweisen.

### Dartellung der Erfindung und bevorzugte Ausführungsformen

Ein typischer Antikörper, beispielsweise vom IgG Typ, besteht aus zwei leichten Polypeptidketten (L-Ketten) und zwei schweren Polypeptidketten (H-Ketten). Diese beiden Ketten sind durch Disulfidbrücken miteinander verbunden. Sowohl die L-Ketten als auch die H-Ketten bestehen aus einer variablen Domäne (V_{L} und V_{H}) am jeweiligen NH₃ - Terminus. Die L-Ketten besitzen eine konstante Domäne (C_{L}), die H-Ketten dagegen weisen drei konstante Domänen auf (C_{H}1 bis C_{H}3) und zusätzlich eine Gelenkregion, welche Cystein Reste haben. Die SH Gruppen dieser Cystein Reste bilden die -S-S- Bindungen aus, welche die beiden einzelnen Monomere des kompletten Antikörperdimers verbinden. Die Spezifität solch eines Antikörpers liegt grundsätzlich in den hypervariablen Regionen der variablen Domäne. Dort befindet sich der sogenannte "antigen - binding site". Kommt ein menschlicher oder allgemein tierischer Organismus in Kontakt mit einem für den jeweiligen Organismus als fremd erkannten Angens, beispielsweise einem infektiösen Erreger, werden spezifische Antikörper bzw. Immunglobuline (IgG, IgM, IgA, lgD und/oder IgE) gegen solch ein Agens gebildet.

Neben den direkten Nachweismethoden, die ein Erfassen des jeweiligen infektiösen Erregers in Blut, Geweben, Exkrementen oder Sekreten nach Auftreten der ersten Krankheitssymptome voraussetzen, spielt die Diagnose über den verläßlichen Antikörpernachweis eine entscheidende Rolle. In vielen Fällen dürfte der Antikörpernachweis die einzige Möglichkeit einer Infektionsbestimmung oder eines Infektionsnachweises darstellen. Dies dürfte insbesondere dann von Bedeutung sein, wenn bei der ersten klinischen Manifestationen die Erreger entweder nur kurzfristig oder gar nicht mehr nachweisbar, okkult oder inapparent sind oder die dafür notwendigen Verfahren sich technisch als sehr schwierig oder differentialdiagnostisch als problematisch erweisen. Beispielsweise kann dies der Fall sein bei Borreliosen, Legionellosen, Lues, Treponematosen, Hepatitis A, Masernviren, Rubellaviren, Meningoenzephalitis, Arboviren, Toxoplasmose, Echinokokkose, Toxocariasis (Spulwurminfektionen bei Tieren) oder manche Endomykosen, um nur einige Beispiele zu nennen.

Es ist von weiterer erheblicher Bedeutung, Infektionen innerhalb eines möglichst kurzen Zeitintervalls zwischen dem Infektionsereignis und dem ersten Auftreten spezifischer Antikörper diagnostisch sicher zu erfassen. Da das Auftreten von insbesondere IgM und IgA auf eine vor kurzer Zeit eingetretenes Infektionsereignis und auf eine akute Infektion hinweisen, kommt insbesondere der Diagnostik von Antikörpern der IgM- und der IgA-Isotypen eine besondere Bedeutung zu.

IgM wird typischerweise unmittelbar dann synthetisiert, wenn der betreffende Organismus einem Antigenstimulus ausgesetzt wird (z.B. durch infektiöse Agentien oder Erreger oder Teilen davon). Daher zeigen erhöhte IgM-Spiegel oder IgM-Titer in der Regel eine kurz vorher eingetretene Infektion, eine erfolgte Antigenexposition oder eine Reaktivierung bei Erregerpersistenz (beispielsweise bei Lues oder Toxoplasmose) an. Somit, da IgM Antikörper schon in frühen Infektionsstadien auftreten, folgt notwendigerweise, daß die Identifizierung oder der verläßliche Nachweis über das Vorhandensein von IgM von wesentlicher diagnostischer Bedeutung für das Früherkennen eines Infektionsereignisses oder einer bereits manifesten Infektionskrankheit oder irgendeiner Antigenexposition ist.

Das Auftreten von IgA läßt ebenfalls auf eine akute, wenn auch mehr lokale Infektion schließen. Das meiste IgA kommt in Sekreten (z.B. Tränenflüssigkeit, Speichel, Schweiß, Schleim, Kolostrum) vor und wird in den entsprechenden Orten synthetisiert (Tränendrüsen, Speicheldrüsen, Gastrointestinaltrakt, Mukosa, Milchdrüsen der laktierenden Brust). Insofern wird dem IgA eine besondere Bedeutung bei der primären immunologischen Abwehr gegen lokale Infektionen zugesprochen, beispielsweise bei Infektionen des Respirations- und Gastrointestinaltrakts oder allgemein bei lokalen Infektionsereignissen.

Trotz der herausragenden Bedeutung dieser beiden genannten Immunglobulin Isotypen für die rasche und frühe Diagnose von insbesondere akuten Infektionsvorgängen, haben die restlichen Immunglobulin Isotypen (insbesondere IgG und IgE) selbstverständlich ebenfalls ihren bekannten Platz in der serologisch-immunologischen Diagnostik. Auch in diesen Fällen ist ein verläßlicher Nachweis über das Vorhandensein oder Fehlen der Antikörper von elementarer Bedeutung.

Betreffend bakterielle Infektionen und die daraufhin synthetisierten Antikörper werden zu deren Nachweis hauptsächlich Bakterienagglutination (WIDAL-Reaktion), Komplement-Bindungsreaktion, indirekte Hämagglutination, Latex-Agglutination, Immunfluoreszenz-Test oder Enzymimmunoassay eingesetzt. Je nach dem gewählten Verfahren können diese Tests zwar erregerspezifische Antikörper der Klassen IgG und IgM erkennen (bei einigen Erregern auch IgA, wenn eine systemische Immunantwort in Gang gesetzt wird), zum Teil können sie jedoch nicht sicher oder überhaupt nicht zwischen IgG- und IgM-Antikörpern differenzieren. Bei manchen Erregern, wie beispielsweise solchen der Gattung Treponema (Treponematosen), nämlich *Treponema pallidum pallidum* (venerische Syphillis), *Treponema pallidum endemicum* (endemische Syphillis), *Treponema pallidum pertenue* (Frambiöse) und *Treponema carateum* (Pinta) ist noch nicht einmal eine serologische Differenzierung untereinander möglich.

Hinsichtlich Virusinfektionen und der aufgrund solcher Infektionen gebildeten Antikörper werden zu deren serologischen Nachweis u. a. die Komplementbindungsreaktion, der Haemagglutinationshemmtest, der Neutralisationstest, der indirekte Immunfluoreszenztest, die passive Haemagglutination, der Enzymlinked Immunoassay (ELISA, EIA), der Radioimmunassay oder der Immunoblot benutzt. Auch in diesem Fall, wie schon für die bakteriellen Infektionen beschrieben, spielt der Nachweis von IgM (aber auch von IgA) die entscheidende Rolle zur serologisch-immunologischen Diagnose akuter Infektionen (DOERR, H. W. et al., Infection 15, 93 - 98, 1987; LINDE, G. A. et al. Infect. Immun. 42., 237 - 244, 1983) oder Rubella Infektionen (LINDE, G. A., J. Clin. Microbiol. 21, 117 - 121, 1985). Die Verläßlichkeit der IgM - Antikörpertests kann von ganz besonderer Bedeutung sein. Beispielsweise kann dies bei Rubella-lnfektionen in Betracht kommen, da im Falle positiver Befunde meist eine Interruptio indiziert sein dürfte.

Auch beispielsweise bei retroviralen Infektionen, insbesondere bei solchen, die durch HIV (Human Immunodeficiency Virus) verursacht werden und schwere Erkrankungen mit Schädigungen des Immunsystems hervorrufen, wie insbesondere AIDS (Acquired Immunodeficiency Syndrome) oder ARC (AIDS - Related Complex), und mit spezifischen Erkrankungen (Tumore und/oder opportunistische Infektionen wie beispielsweise Karposisarkome, Lymphome und/oder Pneumocystis Infektionen) einhergehen, ist die sichere IgM - Diagnostik gleichfalls von großer Bedeutung. Das trifft offenbar insbesondere für solche IgM - Antikörper zu, die niedrigaffin sind (low affinity antibodies) und beispielsweise Epitope auf dem HIV Tat Protein erkennen. In diesem Zusammenhang sei insbesondere auf die dafür relevante Offenbarung in der WO 93/22349 hingewiesen.

Als weiteres Beispiel spielt der Nachweis von spezifischen IgM-Antikörpern gleichfalls eine wichtige Rolle bei der Diagnose der akuten Cytomegalievirus-Infektion. Zum Nachweis von Cytomegalievirus-spezifischen IgM-Antikörpern wurden bekannte und kommerziell erhältliche IgM-ELISA-Techniken miteinander verglichen (WOLFF, M. H. et al., Ärztl. Lab. 30, 357 - 360, 1984). Hierbei hat es sich gezeigt, daß große Diskrepanzen zwischen den erhaltenen Ergebnissen auftraten, sodaß nicht von einer zuverlässigen IgM-Diagnostik bei Cytomegalievirus-lnfektionen gesprochen werden kann.

Auch bei manchen Parasitosen, insbesondere bei Toxoplasmose (z.B. Infektionen mit *Toxoplasma gondii),* kommt dem Nachweis von IgM Antikörpern eine besondere Bedeutung zu, gleichwohl innerhalb der serologischen Diagnostik zunächst auf spezifische Gesamtimmunglobuline untersucht wird. Bei den meisten Parasiteninfektionen sind zirkulierende Antikörper festzustellen, vornehmlich bei solchen Parasiten, die in einen intensiven Kontakt mit dem Immunsystem treten. In vielen Fällen steht der direkte Nachweis des betreffenden Erregers im Vordergrund einer Parasitendiagnostik. Dies gelingt jedoch nicht immer. So ist es bei Toxoplasma-Infektionen unmöglich, die Erreger direkt nachzuweisen, sodaß in solchen Fällen der serologisch-immunologischen Diagnostik eine entscheidende Bedeutung zukommt.

Insbesondere kommt den quantitativen Enzymimmunoassays auf spezifisches IgG und IgM eine wichtige Funktion zu und wegen seiner diagnostischen Sensitivität und Spezifität ist der Enzymimmunoassay auf spezifisches IgM anderen Testsystemen (z.B. indirekter Immunfluoreszenztest) vorzuziehen. Zum Nachweis von spezifischen IgM ist auch der Immunsorbent Agglutination Assay (ISAGA) geeignet (SAATHOFF, M.; SEITZ, H.M., Z. Geburtsh. Perinat. 189, 73 - 78, 1985).

Bei Endomykosen, die synonym u.a. auch als systemische Mykosen, Parenchymmykosen oder tiefe bzw. viszerale Mykosen bezeichnet werden, kommt der Immundiagnostik insofern eine besondere Bedeutung zu, als eine über die Kultivierung erstrebte Absicherung der Diagnose nicht immer möglich ist, da der Infektionsherd nicht sicher lokalisierbar oder einer Exzision nicht zugänglich ist. Somit kann der Nachweis einer immunologischen Auseinandersetzung des infizierten Organismus mit dem Erreger oftmals das früheste und auch einzige labordiagnostische Anzeichen einer Endomykose sein.
Hinsichtlich ihrer Epidemiologie lassen sich die in Frage kommenden Erreger in zwei Gruppen einteilen: i) in die weltweit verbreitete und auch in Europa häufig vorkommenden Erreger der Gruppe der *Candida-Arten, Aspergillus-Arten* und *Cryptococcus neoformans* und ii) in die zweite Gruppe bestehend aus *Coccidoides immitis, Histoplasma capsulatum, Blastomyces dermatitis, Paracoccidioides brasiliensis.* Obwohl die zweite Gruppe vorwiegend auf den amerikanischen Kontinent beschränkt ist und die durch sie verursachten Endomykosen in Europa relativ selten vorkommen, finden sie in letzter Zeit wegen des intensiveren Fernreiseverkehrs verstärkt Beachtung.

Zur Differenzierung von beispielsweise latenten Candida-Infektionen (transiente Fungämie) von manifesten Candida-Mykosen, ist der Verlauf der IgM-Antikörper im indirekten Immunfluoreszenztest bedeutsam.

Bei Aspergillus-Infektionen ist der Nachweis von entsprechenden Antikörpern in Präzipiationsreaktionen von Bedeutung. Zur Überwachung mykosegefährdeter Patienten erscheint der indirekte Hämagglutinationstest als besonders geeignet, da er empfindlich auf IgM-Antikörper reagiert.

Obwohl demzufolge seit Jahren die serologisch-immunologische Diagnostik mit Nachweis der jeweiligen Antikörper eine breite Anwendung insbesondere bei infektiösen Erkrankungen der verschiedensten Art findet und dafür eine Anzahl Assays und serologische Testkits entwickelt und zur Verfügung stehen, sind diese vielfach wegen ihrer niedrigen Spezifität und ihres geringen Vorhersagewertes nicht sehr zuverlässig (HOEK, F.J. et al., J. Clin. Microbiol. 30, 1525 - 1528, 1992).
Auf der anderen Seite wurden eine Reihe an Verfahren entwickelt, die viele Schritte umfassen, technisch und zeitlich sehr aufwendig und zudem teuer sind. Dies gilt sowohl für diagnostische Verfahren bei bakteriellen Infektionen (HOEK, F. J. et al. 1992, loc. cit.; LIM, P. L. & KO, K. H., J. Immunol. Methods 135, 9 - 14, 1990) als auch bei Virusinfektionen (MAYO, D. R., et al., J. Clin. Microbiol. 29, 2865 - 2867, 1991).

Bis in jüngster Zeit wurden Verfahren vorgeschlagen, mit dem Ziel, eine zuverlässige Diagnostik auf Basis eines Antikörpernachweises bereitzustellen. Solche Verfahren dürften jedoch aufgrund ihres technischen, materiellen und/oder zeitlichen Aufwandes wenig praktikabel sein oder sie machen eine schnelle und unkomplizierte Handhabung unmöglich. Es wurde beispielsweise ein kombinierter ELISA vorgeschlagen, der jedoch aus verschiedenen Schritten aufgebaut ist (Coating, Waschen, Plasmainkubation, Inkubation Peroxidase-markierter Antikörper, erneutes Waschen, Messung der Enzymaktivität) und zudem einen vergleichsweise aufwendigen technischen und apparativen Aufwand benötigt (FOMSGAARD, A. & DINESEN, B., 1987, *loc, cit.).* Für eine schnelle diagnostische Verwendung ist ein solches Verfahren nicht geeignet und das bestehende Problem einer fehlenden Immunglobulin-Kontrolle wird dadurch ebenfalls nicht gelöst, wie auch nicht durch die anderen oben beschriebenen diagnostischen Verfahren.

Aber selbst die Verwendung modernerer und aufwendigerer Enzym-Immunoassays für Antikörper konnten die Probleme der Standardisierung bei der Antikörperbestimmung bisher nicht lösen. So wird es allgemein als großer Nachteil beschrieben, daß nur sehr begrenzt Referenzpräparationen zur Verfügung stehen, die eine Kontrolle und ein Vergleich der erhaltenen Resultate, insbesondere zwischen verschiedenen Laboratorien, schwierig oder unmöglich gestalten und eher zur Konfusion beitragen (MALVANO, R. et al., In: Methods of enzymatic analysis, Bergmeyer, H. U., ed., VCH Verlagsgesellschaft, Weinheim, 1986, S. 2 - 10)

Als ein jedoch sehr gravierender Nachteil bei allen bisher bekannten Verfahren zur Immundiagnostik stellt sich häufig das Fehlen eines Kontrollserums oder eines spezifischen standardisierten Produkts (IgX - Kontrolle) heraus. Die daraus entstehende Hauptproblematik eines mangelhaft sicheren Nachweises von beispielsweise IgG oder IgM Antikörpern und völlig unzureichenden, da nicht vergleichbaren, quantitativen Ergebnissen (MALVANO, R. et al. In: Bergmeyer, H.U., Ed., Methods of enzymatic analysis. Vol X, 2 - 10, 1986), läßt keine zuverlässige Immundiagnostik bei Infektionen, beispielsweise bakterieller Art, zu (HILL, H. R. & MATSEN, J. M., J. Infect. Dis. 147, 258 - 263, 1983).
Ein ganz besonderer Nachteil liegt allen bekannten serologisch-immunologischen Diagnoseverfahren insofern zugrunde, als daß es nicht oder nur sehr unzureichend möglich ist, das jeweils verwendete Testsystem hinsichtlich seiner tatsächlichen Zuverlässigkeit und seines Funktionierens zu überprüfen, wenn insbesondere ein negatives Ergebnis erhalten wurde. Ein negatives Ergebnis wäre in diesem Sinne, wenn kein IgX, beispielsweise ein IgM, nachgewiesen werden konnte, mit dem daraus gezogenen - möglicherweise falschen und daraufhin fatalen - diagnostischen Schlußfolgerungen. In den meisten Fällen steht zu wenig oder gar kein als positives Kontrollserum geeignetes Patientenserum zur Verfügung, insbesondere bei akuten Infektionsereignissen, sodaß zu Kontrollzwecken nicht auf Patientenseren zurückgegriffen werden kann.

Ein weiterer Nachteil bei den bisher benutzen und verfügbaren Verfahren ist die relative Instabilität der für die immundiagnostischen Tests zu verwendeten Antikörper bzw. Antikörperpräparationen, insbesondere von solchen, die IgM Antikörper betreffen. Dieser allgemein bekannte Nachteil der Instabilität von Antikörperpräparationen liegt insbesondere darin, daß beispielsweise IgM bei niedrigen Temparaturen (+ 4°C) und selbst in gefrorenem Zustand rasch abgebaut wird.

Sowohl die aus je einem Antikörpermolekül bestehenden und insbesondere durch gentechnische Maßnahmen veränderte und hergestellte chimären Antikörper als auch die bisher beschriebenen durch Linkermolekülen verbundenen Antikörperkonjugate sind nicht in der Lage, die bestehenden und oben beschriebenen Nachteile zu beseitigen

Demzufolge besteht ein dringendes und nach wie vor unbefriedigtes Bedürfnis an einem Mittel für die zuverlässige serologisch-immunologische Diagnostik, welches ein sicheres und schnelles Diagnostizieren und Nachprüfen von durch die bekannten Diagnoseassays erhaltenen Resultaten. Insbesondere von solchen diagnostisch wertvollen Resultaten, die auf der Grundlage des Nachweises von Immunglobulinen bzw. Antikörpern beruhen, welche von infektiösen Agentien viralen, prokarvotischen und eukaryotischen Ursprungs abstammen, sowohl im Rahmen von Routinebestimmungen als auch bei komplexeren und technisch aufwendigen Immunoassays.

Aufgabe der vorliegenden Erfindung ist es, die im Stand der Technik auftretenden Probleme bei der serologisch-immunologischen Diagnostik zu lösen und die dargelegten Nachteile zu beseitigen.

Erfindungsgemäß wird diese Aufgabe gelöst durch chimäre Antikörper gemäß Anspruch 1, die dadurch gekennzeichnet sind, daß sie aus einem ersten nicht-humanen Immunglobulin und einem zweiten humanen Immunglobulin bestehen, wobei beide Immunglobuline kovalent miteinander verbunden sind und in diesem Sinn ein Heterodimer bilden gemäß der allgemeinen Formel Y₁-Z-Y₂, worin Y₁ ein erstes Immunglobulin der allgemeinen Formel IgX ist und nicht-humanen Ursprungs ist und worin X für G, M, A, E oder D und deren Isotypen steht, Z eine direkte oder indirekte kovalente Bindung bedeutet und Y₂ ein zweites Immunglobulin der allgemeinen Formel IgX und humanen Ursprungs ist, wobei X die oben beschriebenen Bedeutung hat.

Demzufolge werden in der vorliegenden Erfindung chimäre Antikörper beschrieben und bereitgestellt, bestehend aus einem ersten Immunglobulin, welches aus einem nicht-humanem Säugetierorganismus stammt und einem zweiten Immunglobulin, welches menschlichen Ursprungs ist, wobei das erste Immunglobulin mit dem zweiten Immunglobulin kovalent, direkt oder indirekt, gemäß der allgemeinen Formel Y₁-Z-Y₂, worin Y₁ das besagte erste Immunglobulin und Y₂ das besagte zweite Immunglobulin bedeutet und Z für eine kovalente Bindung steht, miteinander verbunden sind. Die betreffenden ersten und zweiten Immunglobuline Y₁ und Y₂ sind Immunglobuline der IgX-Klasse, in der X die Bedeutung von G, M, A, D, oder E hat. Die erfindungsgemäßen chimären Antikörper finden in der Diagnostik Verwendung und eignen sich in vorteilhafter Weise als diagnostisches Mittel und zur Absicherung von Ergebnissen bei der serologisch-immunologischen Bestimmung oder Diagnose von durch apathogene und pathogene Agentien hervorgerufene Infektionen, insbesondere bei der diagnostischen Bestimmung von infektiösen Erkrankungen bzw. beim Nachweis von Antikörpern in Seren oder anderen Körperflüssigkeiten oder Geweben eines Säugetierorganismus', die aufgrund einer immunogenen oder antigenen Stimulation in dem betroffenen Säugetierorganismus einschließlich des Menschen gebildet werden.

In seiner allgemeinsten Ausführungsform lassen sich die erfindungsgemäßen chimären Antikörper in vorteilhafter Weise zur Absicherung von Resultaten bei der Diagnose oder Bestimmung bzw. beim Nachweis von solchen Immunglobulinen bzw. Antikörpern verwenden, die aufgrund einer Reaktion eines Säugetierorganismus' mit einem Immunogen oder Antigen gebildet werden. Die erfindungsgemäßen chimären Antikörper können auch selbst in vorteilhafter Weise zur serologisch-immunologischen Diagnostik zum Zwecke der Bestimmung und des Nachweises der genannten Immunglobuline bzw. Antikörper verwendet werden.

Die erfindungsgemäßen chimären Antikörper lassen sich somit in vorteilhafter Weise bei der serologisch-immunologischen Diagnostik von solchen Infektionen verwenden, die durch virale, prokaryotische oder eukaryotische Strukturen, Zellen oder Organismen, insbesondere durch Bakterien, Viren, Mykosen (Endomykosen) oder Parasiten (Parasitosen), verursacht werden.

Die Herstellung der erfindungsgemäßen chimären Antikörper kann nach den bekannten Methoden zur Gewinnung von hybriden Proteinen erfolgen, welche proteinchemische oder gentechnische Methoden oder linkertechnologische Verfahren umfassen. Bevorzugt wird die Verwendung von Linkermolekülen.

Speziellere Ausgestaltungen der erfindungsgemäßen Antikörper, deren Verwendung und Verfahren zu deren Herstellung sind gemäß den weiteren Unteransprüchen und den verschiedenen Anspruchskategorien aufgezeigt.

Vorteilhafterweise wird die Aufgabe durch einen chimären Antikörper der allgemeinen Formel Y₁-Z-Y₂ gelöst, in dem Y₁ ein hochaffines IgX mit bekannter Spezifität ist und Y₂ ein IgX mit unbekannter und von Y₁ verschiedener Spezifität ist, wobei X und Z die oben angegebene Bedeutung hat.

Bevorzugt werden jedoch solche chimäre Antikörper, worin gemäß der allgemeinen Formel Y₁-Z-Y₂ mit der vorgenannten Definition der Term Z die Bedeutung einer indirekten kovalenten Bindung hat.

Noch bevorzugter werden solche chimäre Antikörper gemäß der allgemeinen Formel Y₁-Z-Y₂ mit der vorgenannten Definition, bei denen Z ein Linkermolekül ist, sodaß das besagte erste Immunglobulin Y₁ mit dem besagten zweiten Immunglobulin Y₂ über Linkermoleküle miteinander kovalent verbunden ist.

Ganz bevorzugt sind solche chimäre Antikörper gemäß der allgemeinen Formel Y₁-Z-Y₂ mit der vorgenannten Definition, bei denen das humane Immunglobulin Y₂ ein IgM ist, welches über das Linkermolekül Z kovalent mit dem spezifischen nicht-humanen Immunglobulin Y₁ mit der Bedeutung IgX, verbunden ist.

Ganz besonders bevorzugt sind solche chimäre Antikörper gemäß der allgemeinen Formel Y₁-Z-Y₂ mit der vorgenannten Definition, bei denen Y₁ Spezifität gegen Spirochaeten, insbesondere der Gattungen Borrelia oder Treponema besitzt.

Überraschenderweise hat es sich gezeigt, daß die erfindungsgemäßen chimären Antikörper bei der serologisch-immunologischen Diagnostik als wertvolles Mittel zum zuverlässigen Überprüfen von negativen Ergebnissen bzw. zum Ausschluß von falsch-negativen Ergebnissen bei beliebigen Diagnoseassays, die zum Zweck eines Antikörpernachweises angewandt werden, besonders geeignet sind. Ein mit diesen Vorteil eng verbundene Eigenschaft der erfindungsgemäßen chimären Antikörper gemäß der allgemeinen Formel Y₁-Z-Y₂ liegt offensichtlich darin, daß Y₁ als Antigen-spezifisches IgX wirkt, da es aus einem mit einer interessierenden spezifischen immunogenen Sustanz immunisierten Tier abstammt und somit diese immunogene Substanz als Reaktionspartner erkennt, während Y₂ ein aus dem Menschen stammendes IgX ist und von einem anti-lgX erkannt wird, wobei X für Y₁ und Y₂ die oben angegebene Bedeutung bzw. die weiter unten dargelegte Definition für "Immunglobuline" bzw. "Antikörper" hat. Insofern können die erfindungsgemäßen chimären Antikörper als Ersatz für ein spezifisches humanes IgX, beispielsweise IgM, wenn Y₂ = IgM ist, angesehen werden, wonach Y_{1[IgG}]-Z-Y_{2[IgM]} einen spezifischen humanen IgM-Antikörper simuliert mit den genannten unerwarteten Eigenschaften. Demzufolge kann mit einem erfingungsgemäßen chimären Antikörper der allgemeinen Formel Y₁-Z-Y₂ ein Immunoassay überprüft werden, in dem beispielsweise ein mit einem Enzym oder einem Fluoreszenzmarker verbundenes anti-lgX, beispielsweise anti-IgM, woran das zu diagnostizierende IgX, beispielsweise IgM, binden soll, durch einen der erfindungsgemäßen Antikörper der allgemeinen Formel Y₁-Z-Y₂, beispielsweise Y_{1[IgG]}-Z-Y_{2[IgM]}, ersetzt wird.

Als weiterer Vorteil hat es sich herausgestellt, daß die erfindungsgemäßen chimären Antikörper stabiler sind als die herkömmlichen Immunglobulin-Präparationen, insofern sie eine höhere Haltbarkeit aufweisen und noch nach monatelanger Lagerung in der Kälte keine Beeinträchtigung in ihrer Funktion als diagnostisches Mittel zeigen. In der Regel sind Immunglobuline, insbesondere IgM, trotz Aufbewahrung in der Kälte einem proteolytischen Abbau ausgesetzt, der keine Aufbewahrung über einen längeren Zeitraum als ein bis zwei Wochen gestattet. Die Ursache dieses festgestellten Vorteils, insbesondere bei solchen chimären Antikörpern, bei denen Z in der allgemeinen Formel Y₁-Z-Y₂ die Bedeutung eines Linkermoleküls hat, ist nicht genau bekannt.
Ferner liegt ein zusätzlicher Vorteil der erfindungsgemäßen chimären Antikörper darin, daß große Mengen an einem für die serologisch-immunologische Diagnostik wichtigen Mittel im homogener Form zur Verfügung gestellt werden kann, welches nunmehr in vergleichbarer Qualität und mit vergleichbaren Eigenschaften vorliegt.

Neben diesen vorteilhaften und überraschenden Effekten besitzen die erfindungsgemäßen chimären Antikörper außerdem eine bessere Bindungsfähigkeit.
Diese Bindungsfähigkeit der erfindungsgemäßen chimären Antikörper wird hauptsächlich dadurch bedingt, weil sie aus einem hoch affinen IgX, entsprechend Y₁ in der allgemeinen Formel Y₁-Z-Y₂, bestehen.

Die erfindungsgemäßen chimären Antikörper können bei allen bekannten serologisch-immunologischen diagnostischen Verfahren verwendet werden. Eine bevorzugte Verwendung liegt bei solchen immundiagnostischen Verfahren, bei denen die gewünschten Reaktionen auf Basis von IgM beruhen, da in diesen Fällen eine vor kurzem eingetretene Antigenexposition oder ein akutes Infektionsereignis angezeigt wird und weil hierbei ganz besonders deutlich die Nachteile des Fehlens einer Immunglobulin-Kontrolle (kein IgM-Kontrollserum, kein standardisiertes Produkt) bzw. das Auftreten fehlerhafter, nicht vergleichbarer Testergebnisse und Befunde zu Tage treten und falsch-negative Ergebnisse besonders schwerwiegende Folgen für den betreffenden Patienten haben können.

Insbesondere eignen sich die erfindungsgemäßen chimären Antikörper bei Immunfluoreszenztests, beispielsweise direkter (IFT) oder indirekter Immunfluoreszenztest (IIFT); Enzymimmunoassays (EIA); Agglutinationstests, wie beispielsweise Agglutinationshemmtest, Immunsorbent Agglutination Assay (ISAGA); Radioimmunassays (RIA); Immunoradiometrischer Assay (IRMA), Enzyme Linked Immunosorbent Assay (ELISA), µ-capture-Assay; Enzym Membran Immuno Assay (EMIA); Immuno Enzymo-Metric-Assay (IEMA); Double Antibody Liquid Phase-Assay (DALP-Technik):; Fluoreszenzimmunoassay (FIA), beispielsweise homogener Fluoreszenzimunoassay, Fluoreszenz-Polarisations-lmmunoassay, Fluoreszenz-Enhancement-lmmunoassay, Fluoreszenz Quenching Immunoassay, Fluoreszenz Excitation Transfer Immunoassay (FETIA), Substrate Labelled Fluorescent Immunoassay (SLIFA), Heterogener Fluoreszenz Immunoassay, Immunofluorometrischer Assay (IFMA), Time Resolved Fluorescence Immunoassay (TR-FIA); Lumineszenzimmunoassay, beispielsweise Chemilumineszenz Immuno Assay (CELIA), Solid Phase Antigen Luminescence Technique (SPALT), Immunoluminometrischer Assay (ILMA), Immunoluminometric Labelled Second-antibody Assay (ILSA); Immunoblot bzw. Western blotting oder Dot-blot Techniken.
Es soll an dieser Stelle hervorgehoben werden, daß die oben beispielhaft aufgezählten und für die Verwendung der erfindungsgemäßen chimären Antikörper geeigneten Verfahren nicht vollständig sind und auch nicht sein müssen, da derartige Verfahren, die der serologisch-immunologischen Diagnostik zur Verfügung stehen und die auf Basis von Immunglobulinen bzw. Antikörpern arbeiten, dem Fachmann bekannt sind. Überdies sind derartige Verfahren und Techniken in der Literatur vielfältig beschrieben, beispielsweise von BAUER, S., Lab. Med. 3, 50, 1979 (Immunfluoreszenztechniken); DWENGER, A., J. Clin. Chem. Clin. Biochem. 22, 883 - 894, 1984 (RIA); ADDISON, G. M. & HALES, C. N., In: Kirkham, K.E., Hunts, W.M. eds., Radioimmunoassay methods, Livingstone, Edinburgh, 595, 1971 (IRMA); OELLERICH, M., J. Clin. Chem. Clin. Biochem. 22, 895 - 904, 1984 und MIYAI, K., Adv. Clin. Chem. 24, 61 - 110, 1985 und LINKE, R., Mitt. Dt. Ges. Klin. Chem., 1986, 291 (EIA); HEMMILÄ, I., Clin. Chem. 31, 359 - 370, 1985 und WISSER, H., GIT-Labormedizin 1985, 89 (FIA); WEEKS, I. et al. Clin. Science 70, 403 - .408, 1986 und GADOW, A. et al., J. Clin. Chem. Clin. Biochem. 22, 337 - 347, 1984 und WOOD, W.G. et al., J. Clin. Chem. Clin. Biochem. 22, 349 - 356, 1984 (Lumineszenzimmunoassay); BURNETTE, W.N., Anal. Biochem. 112, 195 - 203, 1981, (Western blotting), DESMONTS, G. et al., J. Clin. Microbiol. 14, 486 - 491, 1981 (ISAGA).

In einer weiteren bevorzugten Ausführungsform finden die erfingungsgemäßen chimären Antikörper Verwendung bei Immunoassays auf Basis von ELISA und Western blotting.

In einer ganz bevorzugten Ausführungsform finden die erfindungsgemäßen chimären Antikörper Verwendung beim indirekten Immunfluoreszenztest (IIFT).

Gegenstand der vorliegenden Erfindung sind daher nicht nur die erfindungsgemäßen chimären Antikörper gemäß Anspruch 1 und seinen näheren Ausgestaltungen gemäß der weiteren Ansprüche dieser Kategorie, sondern auch die Verwendung dieser chimären Antikörper in immundiagnostischen Verfahren und Assays, daß heißt im Rahmen der beschriebenen und dem Fachmann bekannten serologisch-immunologischen Testsysteme, Verfahren und Assays, sowie Verfahren zu ihrer Herstellung. Darüberhinaus umfaßt die vorliegende Erfindung ein Verfahren zur Überprüfung eines immundiagnostischen Verfahrens oder eines Immunoassays unter Verwendung eines der erfindungsgemäßen chimären Antikörpers, wobei nicht nur einzelne Antikörper verwendet werden können, sondern auch in Kombination miteinander, sowie diagnostische Mittel, enthaltend mindestens einen der erfindungsgemäßen chimären Antikörper.

Im Sinne der vorliegenden Erfindung sind unter dem Begriff "Immunglobuline" oder synonym dafür "Antikörper", die für Y₁ und Y₂ stehen, mono- und zumindest bispezifische bivalente bzw. polyvalente, poly- und monoklonale Antikörper zu verstehen, insbesondere der Isotypen IgG (IgG₁, IgG₂, IgG₃, IgG₄), IgM, lgA, IgE und lgD und deren Unterklassen und Derivate davon, aber auch solche, die Fragmente davon darstellen und Derivate davon, einschließlich solcher, die die idiotypische Region des Antikörpermoleküls enthalten, insbesondere solche, die die Fv-Region enthalten, beispielsweise die F(ab)₂ und Fab Fragmente, aber auch a priori chimäre Antikörper oder Hybridantikörper mit mindestens zwei Antigen- bzw. Epitopbindungsstellen, oder bispezifische rekombinante Antikörper (beispielsweise Quadrome, Triome), anti-idiotypische Antikörper und solche davon, die chemisch modifiziert wurden und als Derivate dieser Antikörper zu verstehen sind und die entweder über DNA-Rekombination, mittels Hybridomatechnik oder Antikörper-Engineering oder synthetisch oder semisynthetisch nach an sich bekannten Verfahren herstellbar sind und die bekannten und beschriebenen Neutralisations-, Agglutinations-, Bindungs- und Reaktionseigenschaften hinsichtlich der dem Fachmann bekannten und beschriebenen Methoden der serologisch-immunologischen Diagnostik aufweisen.
Aus der vielfältigen Literatur über die Gewinnung von Antikörpern und Derivaten davon sei nur beispielhaft hingewiesen auf Arbeiten von KÖHLER, G. & MILSTEIN, C., Nature 256, 495 - 497,1975; BIOCCA, S. et al., EMBO J. 9, 101 - 108, 1990; BIRD, R. E. et al. Science 242, 423 - 426, 1988; BOSS, M. A. et al. Nucl. Acids Res. 12, 3791 - 3806, 1984; BOULIANNE, G. L. et al., Nature 312, 643 - 646, 1984; BUKOVSKY, J. & KENNETT, R. H., Hybridoma 6, 219 - 228, 1987; DIANO, M. et al., Anal. Biochem. 166, 223- 229, 1987; HUSTON, J. S. et al., Proc. Natl. Acad. Sci. USA 85, 5879 - 5883, 1988; JONES, P. T. et al., Nature 321, 522 - 525, 1986; LANGONE, J. J. & VUKANIS, H. V. (Hrsg), Methods Enzymol. 121, Academic Press, London, 1987; MORRISON, S. et al. Proc. Natl. Acad. Sci. USA 81, 6851- 6855, 1984; 01, V. T. & MORRISON, S. L., BioTechniques 4, 214 - 221, 1986; RIECHMANN, L. et al., Nature 332, 323 - 327, 1988; TRAMONTANO, A. et al., Proc. Natl. Acad. Sci. USA 83, 6736 - 6740, 1986; WOOD, C. R. et al., Nature 314, 446 - 449, 1985; BRILES, D. E. & KEARNEY, J. F., Methods in Enzymology 116, 174 - 189, 1985.
Mitumfaßt sind auch humanisierte Antikörper oder CDR-grafted Antikörper, beispielsweise gemäß GÜSSOW, D. & SEEMANN, G., Methods in Enzymology 203, 99 - 121, 1991, oder gemäß EP-A 0239 400, WO 92/05274 oder WO 92/15683.

Hinsichtlich der Herstellung von vorzugsweise solchen polyklonalen Antikörpern, die die Komponente Y₁ in der allgemeinen Formel Y₁-Z-Y₂ darstellen, stehen ebenfalls eine Anzahl bekannter Verfahren zur Verfügung. Es können z.B. für diesen Zweck in an sich bekannter Weise verschiedene Tiere durch Injektion mit einem geeigneten bzw. gewünschten Antigen, welches natürlichen Ursprungs, über DNA-Rekombination oder synthetisch bzw. semisynthetisch hergestellt sein kann, oder Teilen davon, immunisiert werden und aus den danach gewonnenen Seren bzw. Blutproben die gewünschten polyklonalen Antikörper nach an sich bekannten Methoden gewonnen und gereinigt werden. Bevorzugte Antigene sind solche, die von Viren, Prokaryonten und Eukaryonten abstammen bzw. solche darstellen. Die Methoden der Herstellung polyklonaler Antikörper beispielsweise über Immunisierung eines Tieres sind dem Fachmann bekannt und sind beispielsweise beschrieben in "Practical Immunology", Hudson, L. & Hay F. C. (eds.), Third Edition, Blackwell Scientific Publications, 1989.
Als Alternative können auch intakte Zellen benutzt werden. Verschiedene Adjuvantien zur Erhöhung der gewünschten Immunantwort auf die Antigen Exposition können, abhängig von dem für die Immunisierung ausgewählten Tier, ebenfalls verwendet werden - beispielsweise Freund's Adjuvant, Mineralgele wie z.B. Aluminiumhydroxid, oberflächenaktive Substanzen wie z.B. Polyanionen, Peptide, Ölemulsionen, Hemocyanine, Dinitrophenol oder Lysolecthin.

Die monoklonalen Antikörper, aus denen die erfindungsgemäßen chimären Antikörper zusammengesetzt sein können, können durch jede beliebige, bekannte Technik erhalten werden, die für die Herstellung von Antikörpern über Kultivierung von Zellinien zur Verfügung stehen. Zu derartigen bekannten Techniken zählen beispielsweise die von KÖHLER, G. & MILSTEIN C., 1975, loc, cit., oder TAGGART & SAMLOF, Science 219, 1228 - 1230, 1983, beschriebenen Verfahren mit Hybridomazellen oder solche mit B Zell Hybridomen (KOZBOR et al. Immunology Today 4, 72 - 79, 1983) oder mittels Hybrid Hybridoma (TAKAHASHI, M et al., Methods in Enzymology, 203, 312 - 327, 1991).
Aber auch über DNA-Rekombination hergestellte Antikörper (sog. Engineered Antibodies) werden im Rahmen der vorliegenden Erfindung mitumfaßt, beispielsweise solche, die nach den beschriebenen Verfahren gemäß COLOMA, M. J. et al., J. Immunol. Methods 152, 89 - 104, 1992; "Methods in Enzymology" 178, 1989 oder EP-A 0 125 023 produziert werden können.

Die nach den bekannten Verfahren herstellbaren und für die erfindungsgemäßen chimären Antikörper verwendbaren Antikörper können nach bekannten Methoden gereinigt werden, beispielsweise über Fällung, Gelfiltration, lonenaustauschchromatographie, Immunoabsorptionschromatographie, Immunoaffinitätschromatographie, HPLC (High Performance Liquid Chromatography) oder Kombinationen davon. Antikörperfragmente, welche den Idiotyp des Moleküls enthalten, können gleichermaßen nach bekannten Verfahren hergestellt werden. Beispielsweise können F(ab')₂ Fragmente durch Pepsin Verdauung des vollständigen poly- oder monoklonalen Antikörpers erhalten werden. Fab Fragmente können erhalten werden, indem beispielsweise die Disulfidbrücken des betreffenden F(ab')₂ Fragments reduziert werden und Fab Fragmente können gewonnen werden beispielsweise durch Behandlung der Antikörpermoleküle mit Papain und gegebenenfalls einem Reduktionsmittel. Derartige Verfahren sind dem Fachmann bekannt und gehören zum Stand der Technik.

Unter dem Begriff "chimäre Antikörper" wird im Rahmen der vorliegenden Erfindung gemäß der allgemeinen Formel Y₁-Z-Y₂ eine Konstruktion verstanden, die aus mindestens zwei intakten Antikörpern besteht, die genuinen Ursprungs sein können oder durch technische Maßnahmen, wie oben beschrieben, manipuliert oder verändert sein können, durch kovalente Bindungen gemäß der allgemeinen Formel Y₁-Z-Y₂ miteinander verbunden sind und ein Hybrid oder Konjugat bzw. Heterodimer bilden, worin ein erster Antikörper Y₁ nicht-humanem Ursprungs und mit bekannter Spezifität und ein anderer zweiter Antikörper Y₂ humanem Ursprungs mit unbekannter oder mit einer von Y₁ verschiedenen Spezifität ist. Darunter fallen aber auch solche Konstruktionen, worin in der allgemeinen Formel Y₁-Z-Y₂ die Symbole Y₁ und Y₂ auch Antikörperfragmente, einschließlich solcher, die die idiotypische Region eines Antikörpermoleküls enthalten, insbesondere die Fv-Region, wie beispielsweise F(ab')₂ oder Fab, bedeuten können, wenn Z ein Linkermolekül oder eine S-S - Bindung ist.

Unter "indirekt" oder "direkt" "kovalent" miteinander verbundenen ersten und zweiten Antikörpern bzw. unter "indirekter" oder "direkter" "kovalenter Bindung" gemäß der Definition von Z in der allgemeinen Formel Y₁-Z-Y₂ ist im Rahmen der vorliegenden Erfindung zu verstehen, daß zwei Antikörper über intermolekulare Bindungen direkt, beispielsweise über S-S - Bindungen, oder indirekt, beispielsweise über Linkermoleküle, miteinander verbunden sind.

Hinsichtlich der bevorzugten erfindungsgemäßen chimären Antikörper, die durch ein Linkermolekül miteinander kovalent verbunden sind, sind im Rahmen der vorliegenden Erfindung alle dem Fachmann bekannten Moleküle oder "Crosslinkers" oder "Cross-Linking-Reagents" zu verstehen, mit denen mindestens zwei gleiche oder verschiedene Proteine oder Peptide nach an sich bekannten Methoden miteinander kovalent verbunden werden können, wobei unter dem Begriff "Crosslinkers" oder "Cross-Linking-Reagents" solche Moleküle mit den genannten Eigenschaften verstanden werden, welche funktionelle Gruppen enthalten, die an Aminosäureresten in Proteinen und Peptiden binden. Demzufolge werden im Rahmen der vorliegenden Erfindung alle Crosslinker bzw. Cross-Linking-Reagentien umfaßt, die intermolekulare kovalente Bindungen zwischen zwei Proteinen oder Teilen davon erzeugen können. Im weiteren wird daher nur noch von "Verbindungsmolekülen" gesprochen, wenn von derartigen Crosslinkern oder Cross-Linking-Reagentien die Rede ist.

Erfindungsgemäß bevorzugt werden insbesondere solche Verbindungsmoleküle, deren reaktive Gruppen gleich (homobifunktionelle Verbindungsmoleküle) oder verschieden (heterobifunktionelle Verbindungsmoleküle) sind.

Aus der Gruppe der homobifunktionellen Verbindungsmoleküle kommen solche in Betracht, die vorzugsweise mit primären Aminen (aminreaktiv) oder mit Sulfhydrylgruppen (thiolreaktiv) reagieren, beispielsweise Imidoester bzw. Maleimide, Alkylhalide, Arylhalide, α-Haloacyle, Pyridyldisulfide.

Bei den heterobifunktionellen Verbindungsmolekülen, die mindestens zwei verschiedene reaktive Gruppen besitzen, sind insbesondere solche geeignet, die eine aminoreaktive funktionelle Gruppe an einem Ende und eine sulfhydrylreaktive Gruppe am anderen Ende besitzen, beispielsweise solche, die als eine erste Gruppe eine N-hydroxysuccinimidester Gruppe und als eine zweite funktionelle Gruppe eine Maleimidgruppe besitzen.
Aber auch solche heterobifunktionelle Verbindungsmoleküle kommen in Betracht, die mit Carboxylgruppen reagieren, beispielsweise mit Glutaminsäure- oder Asparaginsäureresten als Reaktanten. Beispielsweise seien Carbodiimide genannt.
Darüberhinaus sind aber auch die bekannten photoreaktiven Verbindungsmoleküle geeignet, welche eine funktionelle photoaffine Gruppe aufweisen, die erst durch Exposition mit UV-Licht oder anderer sichbarer Strahlung (Photonen) reaktiv werden, beispielsweise aromatische Azide (Arylazide) oder Benzophenone, welche mit Aminen, Sulfhydrylen, Carbohydraten und Carbonylen reagieren.

Beispielsweise eignen sich für die Herstellung der erfindungsgemäßen chimären Antikörper die folgenden Verbindungsmoleküle:
Dithio*bis*(succinimidylpropionat),
3,3'-Dithio*bis*(sulfosuccinimidylpropionat),
Bis(sulfosuccinimidyl)suberat,
Disuccinimidylsuberat,
Disuccinimidyltartrat,
Disulfosuccinimidyltartrat,
Ethylenglycol*bis*(sulfosuccinimidylsuccinat),
*Bis*[2-(succinimidooxycarbonyloxy)ethyl]sulfon,
*Bis*[2-(sulfosuccinimidooxycarbonyloxy)ethyl]sulfon,
Ethylenglycol*bis*(succinimidylsuccinat),
*Bis*maleimidohexan,
1,5-Difluoro-2,4-dinitrobenzol,
Dimethyladipimidat,
Dimethylpimelimidat,
Dimethylsuberimidat,
Dimethyl 3,3'-dithio*bis*propionimidat,
*N*-Hydroxysulfosuccinimid,
1-Etyl-3-(3-Dimethylaminopropyl)-carbodiimid,
4-succinimidyloxycarbonyl-α-methyl-α(2-pyridyldithio)-toluol,
*N*-succinimiyl3-(2-pyridyldithio)propionat,
Sulfosuccinimidyl 6-[3-(2-pyridyldithio)propionamido]hexanoat,
Succinimidyl6-[3-(2-pyridyldithio)propionamido]hexanoat,
Sulfosuccinimidyl4-(*N*-maleimidomethyl)cyclohexan-1-carboxylat,
Succinimidyl4-(*N*-maleimidomethyl)cyclohexan-1-carboxylat,
Sulfosuccinimidyl 2-(7-azido-4-methylcoumarin-3-acetamid)ethyl-1,3'-dithiopropionat,
Sulfosuccinimidyl7-azido-4-methylcoumarin-3-acetat,
Sulfosuccinimidyl6-[α-methyl-α-(2-pyridyldithio)toluamido]hexanoat,
1,4-Di-[3'-(2'-pyridyldithio)propionamido]butan,
*m*-Maleimidobenzoyl-N-hydroxysuccinimidester,
*m*-Maleimidobenzoyl-N-hydroxysuldosuccinimidester,
*N*-Succinimidyl(4-iodoecetyl)aminobenzoat,
Sulfosuccinimidyl(4-iodoacetyl)aminobenzoat,
Succinimidyl4-(*p*-maleimidophenyl)butyrat,
Sulfosuccinimidyl4-(*p*-maleimidophenyl)butyrat,
Azidobenzoyl Hydrazid,
*N*-Hydroxysuccinimidyl-4-azidosalicylsäure,
Sulfosuccinimidyl 2-(*p*-azidosalicylamido)ethyl-1,3'-dithiopropionat,
*N*-[4-(*p*-azidosalicylamido)butyl]-3'(2'-pyridyldithio)propionamid,
*Bis*-[β-(4-azidosalicylamido)ethyl]disulfid,
*N*-hydroxysuccinimidyl-4-azidobenzoat,
*N*-Succinimidyl-6(4'-azido-2'-nitrophenyl-amino)hexanoat,
Sulfosuccinimidyl-6(4'-azido-2'-nitrophenylamino)hexanoat,
*p*-Azidophenylglyoxalmonohydrat,
*N*-5-Azido-2-nitrobenzoyloxysuccinimid,
Sulfosuccinimidyl2-(*m*-azido-o-nitrobenzamido)-ethyl-1,3'dithiopropionat,
*p*-Nitrophenyl-2-diazo-3-3-3-trifluoropropionat,
Sulfosuccinimidyl(4-azidophenyldithio)propionat),
*N*-Succinimidyl(4-azidophenyl)1,3'-dithiopropionate,
*N*-Hydroxysulfosuccinimidyl4-azidobenzoat,
Sulfosuccinimidyl4-(*p*-azidophenyl)butyrat,
1-(*p*-Azidosalicylamido)-4-(iodoacetamido)butan,
4-(*p*-Azidosalicylamido)butylamin,
Sulfosuccinimidyl 7-azido-4-methylcoumarin-3-acetat,
Sulfosuccinimidyl2-(7-azido-4-methylcoumarin-3-acetamid)ethyl-1,3'dithiopropionat,
*N*-(4-azidobenzoylglycyl)-S-(2-thiopyridyl)cystein,
*N,N*'-bis(4-azidobenzoyl)cystine,
2-lminothiolan,
1-[*N*-(2-hydroxy-5-azidobenzoyl)-2-aminoethyl]-4-(*N*-hydroxysuccinimidyl)-succinat,
*N*-[-4-(*p*-azidophenylazo)benzoyl]-3-aminopropyl-*N*'-oxysulphosuccinimidester,
Ethyl-4-azidophenyl-1,4-dithiobutyrimidat,
*N*-(4-azidophenyl)phthalimid,
Glutaraldehyd,
Glutardialdehyd,
Tartryldiazide,
Tartryldi(glycylazide),
Tartryldi(ε-aminocaproylazid) oder
*N,N*'-Bis(3-succinimidyloxycarbonyl)tartaramid.

Die Verwendung solcher Verbindungsmoleküle zur Herstellung der erfindungsgemäßen chimären Antikörper gestatten somit auch vielfache Markierungsmöglichkeiten der erhaltenen Produkte, wie beispielsweise Radiomarkierungen mit radioaktiven Isotopen zum Beispiel über entsprechende lodierung von beispielsweise 1-(*p*-Azidosalycylamido)-4-(iodoacetamido)butan oder beispielsweise Fluoreszenzmarkierungen durch Verwendung geeigneter Verbindungsmoleküle, zum Beispiel von Sulfosuccinimidyl7-azido-4-methylcoumarin-3-acetat. Insofern sind auch solche chimäre Antikörper mitumfaßt, die eine Markersubstanz enthalten oder tragen.

Die genannten Verbindungsmoleküle lassen sich nach an sich bekannten Methoden herstellen und sind kommerziell erhältlich (z.B. bei Pierce Europe B.V., Oud-Beijerland, Holland). Die genannten und weitere für die vorliegende Erfindung geeignete und zu verwendende homo- und heterobifunktionelle Verbindungsmoleküle und deren Anwendungen und Reaktionen mit den reaktiven Gruppen der betreffenden Proteine bzw. Immunglobuline sind ferner individuell beschrieben von BRINKLEY, M., Bioconjugate Chem., 3, 2 - 13, 1992 und von MATTSON, G. et al., Mol. Biol. Rep. 17, 167 - 183, 1993. Weitere zum Zweck der Herstellung der erfindungsgemäßen chimären Antikörper geeignete Verbindungsmoleküle sind individuell beschrieben von SCHEIDTMANN; K. H. In: Protein structure, a practical approach, Creighton, T. E. (Ed.), IRL Press at Oxford University Press, Chapter 4, 93 - 115, 1989; TRAUT, R. R. et al. In: Protein function, a practical approach, Creighton, T. E. (Ed.), IRL Press at Oxford University Press, Chapter 5, 101 - 133, 1989; TAE, H., Methods in Enzymology 91, 580 - 609, 1983; BÄUMERT, H. G. & FASOLD, H., Methods in Enzymology 172, 584 - 609, 1989.

Die Herstellung der über die oben beschriebenen Verbindungsmoleküle kovalent verbundenen erfindungsgemäßen Antikörper kann nach literaturbekannten Methoden erfolgen, wie sie zum Beispiel in den schon genannten Veröffentlichungen über die Verbindungsmoleküle beschrieben sind, einschließlich der dort angegebenen weiteren Literatur (beispielsweise BRINKLEY, M., 1992, loc. cit.; MATTSON, G. et al., 1993, loc. cit.; Katalog von Pierce).
Beispielsweise kann die Herstellung eines erfindungsgemäßen chimären Antikörpers durch kovalente Bindung zwischen Y₁ und Y₂, wobei dafür IgX steht und X die oben genannte Bedeutung hat, gemäß der allgemeinen Formel Y₁-X-Y₂ mit der genannten Definition von Z dadurch erfolgen, daß geeignete Immunglobuline nach an sich bekannter Methode gewonnen und gereinigt werden. Anschließend werden diese Immunglobuline, beispielsweise ein spezifisches nicht-humanes IgG und ein unspezifisches human IgM, gleichbedeutend mit Y₁ beziehungsweise Y₂ in der allgemeinen Formel Y₁-Z-Y₂, mit einem geeigneten Verbindungsmolekül, vorzugsweise ein homobifunktionelles Verbindungsmolekül, insbesondere ein Imidoester, ganz besonders ein Bisimidat, beispielsweise Dimethylsuberimidat, unter geeigneten Bedingungen in Reaktion gebracht. Die Reaktionsprodukte können dann nach bekannten Verfahren von den übrigen Bestandteilen der Reaktionslösung getrennt und gereinigt werden, beispielsweise durch Dialyse, Zentrifugation und/oder Chromatographie.

Bevorzugte Verbindungsmoleküle, die zur Herstellung der erfindungsgemäßen chimären Antikörper der allgemeinen Formel Y₁-Z-Y₂, mit den genannten Bedeutungen für Y₁ und Y₂, geeignet sind und die als Bestandteil Z in den erfindungsgemäßen chimären Antikörpern vorliegen, sind solche aus der Gruppe der homobifunktionellen Verbindungsmoleküle. Ein chimärer Antikörper, der ein solches Verbindungsmoleküle enthält, ist daher bevorzugt. Gleichsam bevorzugt ist die Verwendung derartiger Antikörper für die serologisch-immunologische Diagnostik im oben beschriebenen Sinn.

Ganz bevorzugt ist ein chimärer Antikörper der allgemeinen Formel Y₁-Z-Y₂, bei dem ein erster nicht-humaner Antikörper Y₁ und ein zweiter humaner Antikörper Y₂, mit den genannten Bedeutungen von Y₁ und Y₂, durch Reaktion mit einem Amin-spezifischen homobifunktionellen Verbindungsmolekül, bevorzugt mit einem Imidoester, insbesondere mit einem Bisimidat, ganz besonders mit Dimethylsuberimidat konjugiert bzw. kovalent verbunden ist. Ganz bevorzugt ist dementsprechend die Verwendung derartiger Antikörper für die serologisch-immunologische Diagnostik im oben beschriebenen Sinn.

Ganz besonders bevorzugt ist ein chimärer Antikörper der allgemeinen Formel Y₁-Z-Y₂, bei dem der erste nicht-humane Antikörper Y₁ ein IgG Antikörper ist und der zweite humane Antikörper Y₂ ein IgM Antikörper ist, mit den genannten jeweiligen Spezifitäten, wobei beide über Z, das als Verbindungsmolekül die Bedeutung von Dimethylsuberimidat hat, miteinander kovalent verbunden sind. Daher ist auch die Verwendung eines solchen chimären Antikörpers gemäß der Formel Y_{1[IgG]}-Z-Y_{2[IgM]} für die serologisch-immunologische Diagnostik im oben beschriebenen Sinn ganz besonders bevorzugt.

Die Bereitstellung der erfindungsgemäßen chimären Antikörper kann aber auch dadurch erfolgen, daß, wie oben schon erwähnt, eine direkte kovalente Bindung Z gemäß der allgemeinen Formel Y₁-Z-Y₂ gewählt werden kann. Dies kann insbesondere durch genetische Manipulation bzw. DNA-Rekombination erreicht werden. Beispielsweise dadurch, daß die Eigenschaft zweier Sulfhydryl Gruppen von Cysteinresten genutzt wird, intermolekulare Disulfidbindungen auszubilden. Die dem Fachmann bekannten Methoden der DNA-Rekombination können beispielsweise dazu benutzt werden, neue Cysteinreste an zwei Immunglobulinmolekülen von Interesse derart einzufügen, daß die beiden entsprechenden Monomere zu einem Dimer gemäß der allgemeinen Formel Y₁-Z-Y₂ verbunden werden, worin in diesem Fall Z eine kovalente Bindung im Sinne einer S-S - Bindung wäre (TRAUT, R. R. et al. In: Protein function, a practical approach, Creighton, T. E. (Ed.), IRL Press at Oxford University Press, Chapter 5, 101 - 133, 1989). In der W089/01782 werden beispielsweise veränderte rekombinante Antikörper offenbart, bei denen eine Aminosäure im Antikörpermolekül durch ein Cysteinrest ersetzt ist.

Die Herstellung eines nicht-humanen Antikörpers für die erfindungsgemäßen chimären Antikörper der allgemeinen Formel Y₁-Z-Y₂ kann nach an sich bekannten Methoden erfolgen. Beispielsweise kann ein für die Immunisierung und die darauffolgende Gewinnung und Reinigung eines nicht-humanen Antikörpers geeignetes Tier (beispielsweise Maus, Ratte, Meerschweinchen, Kaninchen, Schaf, Schwein, Huhn) dadurch immunisiert werden, daß es über die bekannten Applikationswege, beispielsweise die intradermale, intramuskuläre, intraperitoneale, intravaskuläre, intracraniale oder subcutane Route die für die diagnostische Untersuchung entsprechend interessante immunogene Substanz per injectionem appliziert bekommt. Als immunogene Substanzen kommen jene in Frage, die in dem betreffenden Organismus eine Immunantwort auslösen bzw. immunogen sind und die mit den Produkten einer Immunantwort, den Antikörpern, reagieren. Insofern werden im weiteren die Begriffe Immunogen und Antigen synonym verwendet. Der Fachmann weiß, wie und auf welche Weise er die Immunisierung durch Applikation des entsprechenden Antigens durchführt.
Die Herstellung eines Antikörpers einer gewünschten Immunglobulinklasse, beispielsweise IgG, in einem geeigneten Tier, beispielsweise einem Kaninchen, kann dadurch erfolgen, daß eine entsprechende Antigenpräparation, beispielsweise eine Bakteriensuspension, gegebenenfalls zusammen mit einem Adjuvans, beispielsweise komplettes Freund-Adjuvans, nach bekannter Methode injiziert wird. Derartige Injektionen können mehrmals hintereinander in im allgemeinen täglichen, wöchentlichen oder monatlichen Intervallen erfolgen.
Die Anzahl der Immunisierungen und die Menge des zu applizierenden Antigens bestimmt maßgeblich die Ausbeute der gewünschten Antikörper im Serum des betreffenden Tieres. Im allgemeinen kann davon ausgegangen werden, daß beispielsweise bei der Immunisierung eines ausgewachsenen Kaninchens eine Gesamtdosis von 0.1 - 5.0 mg Bakterien in Suspension intraperitoneal oder intravenös in einem Volumen von jeweils 0.5 bis 2.0 ml pro Injektion gegeben wird, wobei jede Einzeldosis 0.01 bis 0.1 mg an Bakterienmasse enthalten kann. Auch bei der Aufeinanderfolge der einzelnen Injektionen gibt es keine strengen Regeln, sondern der Fachmann weiß, wie oft und innerhalb welcher Zeitintervalle er die Antigene, die entweder als Proteinlösungen oder als Zell- bzw. Partikelsuspensionen vorliegen können, dem Tier verabfolgen muß.
Bewährt hat sich sowohl bei Proteinlösungen als auch bei Partikel- oder Zellsuspensionen (beispielsweise Bakterien) eine mehrmalige Injektion mit beispielsweise kompletten Freund-Adjuvans unter Verwendung eines Einzelvolumens von ungefähr 0.5 - 2.0 ml, vorzugsweise 0.5 - 1.0 ml pro Tier (beispielsweise Kaninchen). Beim Kaninchen kann beispielsweise davon ausgegangen werden, daß 0.1 bis 1.0 mg an Bakterien, insbesondere 0.5 mg, als Gesamtdosis verabreicht werden, wobei die Einzeldosen in einem Volumen von 0.5 bis 2.0 ml, vorzugsweise 1.0 ml, intracutan, subcutan, intramuskulär oder intravenös, beispielsweise in die Ohrvene, gegeben werden kann. Aber auch andere Verfahren zur Herstellung von nicht-humanen Antikörpern mit oder ohne Zusätze bzw. Immunverstärker, wie z.B. Aluminiumhydroxid, sind dem Fachman bekannt (HUDSON, L. & HAY, F. C., Eds., Practical Immunology, Blackwell Scientific Publications, 3. Edition, 1989).

Die Herstellung eines humanen Antikörpers als zweites Immunglobulin Y₂ als Bestandteil des erfindungsgemäßen chimären Antikörpers gemäß der Formel Y₂-Z-Y₂ kann nach an sich bekannten Standardverfahren erfolgen. Im allgemeinen wird davon ausgegangen, daß aus dem Blutserum von einem Menschen, der eine besonders hohe und pathologische Immunglobulinsyntheserate eines bestimmten Antikörpers IgX aufweist, nach bekannten Verfahren ein gewünschter humaner Antikörper gewonnen werden kann. Insbesondere kommen dafür Patienten in Frage, die an Makroglobulinämie leiden.
Insbesondere kann von einem Menschen, der einen besonders hohen IgM-Spiegel aufweist, beispielsweise von einem Patienten mit Morbus Waldenström bzw. IgM-Plasmozytom, von dem bekannt ist, daß das Blut eines solchen Menschen eine IgM-Konzentration bis zu 100 mg/ml haben kann, das in seinem Blut enthaltene IgM nach Standardmethoden oder beispielsweise über Plasmapherese gewonnen werden.

Die Reinigung des nicht-humanen und des humanen Immunglobulins kann nach den bekannten Verfahren der Salzfällung (beispielsweise mit Ammoniumsulfat) oder der Chromatographie, beispielsweise über Anion-Austauscher (beispielsweise DEAE-DE52, Serva) oder Gel-Chromatographie (beispielsweise Sephacryl, Pharmacia) erfolgen.

Hinsichtlich des der vorliegenden Erfindung zugrundeliegenden Begriffs Immunogen bzw. Antigen werden alle diejenigen Substanzen umfaßt, die in einem Säugetierorganismus eine Immunantwort und somit eine Immunglobulin- (Antikörper-)synthese induzieren können. Insofern kann ein solches Antigen natürlichen Ursprungs sein, über DNA-Rekombination oder synthetisch oder semisynthetisch hergestellt worden sein oder Teile von diesen darstellen. Derartige Antigene können in biologischen Flüssigkeiten eines Säugetierorganismus einschließlich des Menschen extrazellulär, intrazellulär, transzellulär (third space) oder interstitiell vorkommen, wie beispielsweise Blut, Serum und Fraktionen davon, Exkremente (wie z.B. Urin, Faeces), Tränenflüssigkeit, Speichel, Amnionflüssigkeit, Gewebeflüssigkeit, Aszites, Samenflüssigkeit, Pleuraflüssigkeit, Cystenflüssigkeit, Cerebralflüssigkeit, Eiter, Sekrete des Magen-Darm-Trakts, Liquor, Augenkammerflüssigkeit, Ödemflüssigkeit, pathologisch auftretende Flüssigkeiten bei beispielsweise Ileus oder Peritonitis, Sekrete, Zerebrospinalflüssigkeit, Plasma, Lymphe oder Flüssigkeiten, die aus Gewebeextrakten erhalten werden, Gewebe- und Zellkulturen.
Insbesondere kommen solche Antigene in Betracht, die Viren, Prokaryonten und Eukaryonten oder Teile davon sind. Insbesondere Mikroorganismen wie Bakterien (beispielsweise Enterobacterien, Corynebacterien, Spirochaeten insbesondere die Gattungen Treponema und Borrelia, Pseudomonaden, Mycoplasmen, Coccen, Chlamydien, Bacillen), Pilze und Sporen davon (beispielsweise *Candida, Cryptococcus, Blastomyces, Paracoccidoides, Coccidoides, Aspergillus, Histoplasma),* auf DNA- und RNA-Viren beruhende Antigene, beispielsweise virale Capsidantigene oder Hüllproteine (zum Beispiel Hepatitis Viren und verwandte Antigene, wie zum Beispiel HBe, HBc, HBs, Pockenviren, Coxsackieviren, Echoviren, Myxoviren, Rhabdoviren, Togaviren, Reoviren, beispielsweise HIV, Tumorviren, Picornaviren, Herpesviren, Paramyxoviren, Rubellaviren, Mumpsviren, RS-Viren, Coronaviren), Protozoen (wie bespielsweise Leishmania, Trichomonas, Trypanosoma, Sarcocystidae mit Toxoplasma, Eimeriidiae, Plasmodium, Cryptosporiidae), Tumor-spezifische Proteine infizierter oder transformierter Zellen, die im entsprechenden Wirt eine Immunantwort induzieren. Mitumfaßt sind auch über die bekannten Methoden der DNA-Rekombination hergestellten Antigene, beispielsweise wie sie für Toxoplasma gondii in der EP-A 0431541 beschrieben sind, und über die bekannten synthetischen und semisynthetischen Verfahren herstellbaren Antigene. Aber auch Allergene sind hiervon mitumfaßt, da sie ebenso wie die beschriebenen Antigene die Antikörpersynthese in Gang setzen, insbesondere IgE, beispielsweise Milben oder Hausstaub.

Die erfindungsgemäßen chimären Antikörper können in flüssiger oder fester Form vorliegen und in Fläschen, Ampullen oder anderen dazu äquivalenten Behältern, vorzugsweise in solchen aus Glas oder Kunststoffen, gefüllt und verpackt werden.
Hinsichtlich des Vorliegens der Antikörper in flüssiger Form sind die dem Fachmann bekannten Puffer und Medien verwendbar, gegebenenfalls zusammen mit einem Stabilisierungsmittel. Entsprechend eignen sich zum Beispiel Tris-, Phosphat- (beispielsweise Phosphat gepufferte Kochsalzlösung, PBS) oder Carbonat-Puffer, steriles Wasser, einfach- oder bidestilliertes Wasser oder physiologische Kochsalzlösungen. Als Stabilisierungsmittel eignen sich die bekannten und üblichen Zusätze, wie beispielsweise Albumine oder Serumalbumine (Rinderserumalbumin [RSA], Humanserumalbumin [HSA]) oder andere inerte Proteine, aber auch Biozide.
Die erfindungsgemäßen chimären Antikörper können aber auch in lyophilisierter Form vorliegen, wenn sie als festes Produkt bestehen. In solchen Fällen können die dem Fachmann bekannten Techniken der Lyophilisation und der vor der Verwendung der entsprechenden Antikörper durchzuführenden Rekonstitution (beispielsweise mit einem der genannten Puffer und Medien) angewandt werden.
Die erfindungsgemäßen chimären Antikörper liegen entweder in gebrauchsfertiger Verdünnung oder Konzentration vor oder sie können nach Belieben weiter verdünnt oder ankonzentriert werden, wobei der Fachmann weiß, welche individuelle Konzentration oder Verdünnung er abhängig von seinem Verwendungszweck einsetzen möchte. Beispielsweise können die Antikörper in einem Titerbereich von 1 : 20 bis 1 : 640, vorzugsweise 1 : 80 bis 1 : 160 oder um den Bereich von 1 : 100 vorliegen. Zur Verdünnung der Antikörperkonzentration können die oben genannten Puffer und Medien oder sterilen wässrigen Lösungen verwendet werden, beispielsweise PBS, Kochsalzlösungen oder Wasser.
Die erfindungsgemäßen chimären Antikörper können einzeln vorliegen, daß heißt als einziger Bestandteil eines diagnostischen Mittels in Form einer Verpackungseinheit oder sie können als ein Bestandteil unter anderen Bestandteilen in einer Verpackungseinheit vorliegen, zum Beispiel als Kit oder Kit-of-parts.
Ein Kit kann aus einem chimären Antikörper der vorliegenden Erfindung als positive Kontrolle und mindestens einem mit einem bestimmten Antigen beschichteten Objekträger bestehen.
Als Objektträger, auf den die gewünschten Antigene fixiert sind, kommen alle den Fachmann bekannten gebräuchlichen Formen in Frage, insbesondere solche aus transparenten und flächig ausgestalteten Materialien wie zum Beispiel aus Glas oder Kunstoffen (Polyvinyle, Polystyrole, Acrylate).
Ein solcher Kit kann zusätzlich zu diesen beiden Teilen ein IgM spezifisches Antihumanglobulin enthalten, welches gegebenenfalls mit einem Marker, beispielsweise einem Fluoreszenzmarker, wie zum Beispiel Fluoresceinisothiocyanat, verbunden oder konjugiert ist, und/oder ein wie oben schon genannter Puffer (beispielsweise PBS), der als Waschpuffer und/oder zur Serumverdünnung verwendet werden kann.

### Kurze Beschreibung der Figuren

- Fig. 1:: Immunfluoreszenz: das IgG/IgM *Borrelia burgdorferi* (Bb) Hybrid zeigt eine starke spezifische Anfärbung der Borrelien (Verdünnung des Hybrides 1:100), Vergrößerung x400.
- Fig. 2:: Immunfluoreszenz: Anfärbung der Borrelien mit einem IgM Antikörper haltigen human Serum (Verdünnung des Serums 1:20), Vergrößerung x400.

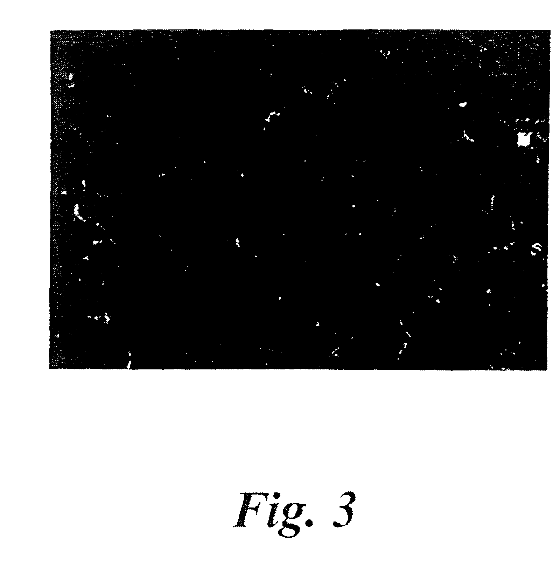

- Fig. 3:: Immunfluoreszenz: wie in Fig. 2, jedoch liegt in diesem Fall ein IgM negatives human Serum vor (Kontrolle), Verdünnung des Serums 1:20. Im Fall der Verwendung eines IgG/IgM Hybrids (wie in Fig. 1), ohne Spezifität für Bb, ist keine Fluoreszenz mehr zu erkennen. Vergrößerung x 400.
- Fig. 4:: Werte einer ELISA Platte: Ordinate - Extinktionswerte der einzelnen Vertiefungen, Abzisse - Verdünnunngsreihen verschiedener Proben, A = 1:100 bis H = 1:12.800 in 2er Verdünnungsstufen. Spalten: S1 = Leerwert, S2 = IgG/IgM Bb Hybrid, S3 = IgG/IgM *Treponema pallidum* (T.pa) Hybrid (zum Vergleich), S4=negatives human Serum, S5=IgG/IgM negativ Hybrid, S6=Probe eines positiven human Serums (IgM), S7 = weitere Probe eines positiven human Serums (IgM).
- Fig. 5:: Westernblotstreifen: A = Molekulargewichtsmarker, B = Amidoschwarz gefärbtes Antigen (Gesamtlysat aus Bb), C = Patienten Serum (enthält IgM Ak gegen 34kD "outer surface protein" [Osp] von Bb), D = nicht Bb spezifisches IgG/IgM Hybrid, E = Bb spezifisches IgG/IgM Hybrid (mehrere Antigene werden erkannt). Antigen: *Borrelia burgdorferi* Stamm B31, Antiserum: IgG/IgM Hybrid Anti Bb.B31 (Verdünnung 1:500), Verdünnung der positiv Kontrolle, negativ Kontrolle 1:100, Verdünnung des Zweitantikörpers Anti-Human-lgM 1:3000.

### Bester Weg zur Ausführung der Erfindung

### Beispiel 1: Herstellung eines IgG:IgM chimären Antikörpers

1.1. Gewinnung von spezifischen nicht-humanem IgG
   1.1.1. Immunisierung
      *Borrelia burgdorferi* Stamm B31 ATCC 35270 wurde nach bekannten Methoden (KRAMPITZ, H. & BARK, S., In: Lyme Borreliosis II, Zbl. Bakt. Suppl. 18 (Stanek, Ed.), Gustav Fischer, Stuttgart, New York, 21 - 25, 1980 und PELZ, K. et al., In: Lyme Borreliosis II, Zbl. Bakt. Suppl. 18 (Stanek, Ed.), Gustav Fischer, Stuttgart, New York, 35 - 39, 1980) isoliert und in 10ml modifiziertes Barbour-Stoenner-Kelly (BSK II)(Barbour, Yale J. Biol. Med. 57, 71, 1984) Medium bei 35°C für 3-4 Tage vermehrt. Adequates Wachstum wurde im Dunkelfeldmikroskop kontrolliert. Die Spirochaeten wurden bei 5.000g abzentrifugiert, 2 Mal in PBS (Phosphat gepuffertes Kochsalz) gewaschen und in 0,5ml PBS resuspendiert. Dieses Suspension wurde mit Freundschen Komplettes Adjuvanz (Sigma, F-5881), Gesamtvolumen 1ml, gemischt und einem Kaninchen intramuskulär verabreicht. Zwei weitere Immunisierungen in 3-4 wöchigen Abständen wurden durchgeführt. 2 Wochen nach der letzten Gabe von Borrelien zeigte das Tier ein Titer von 1:1280 in der Indirekten Immunfluoreszenz (Einzelheiten, siehe 2.1.1. und 2.2.1.) und von 1:20.000 in dem Borrelia burgdorferi Haemagglutinations (BbHA) Test (LD Labordiagnostika, Heiden, Deutschland).
   1.1.2. Reinigung des aus 1.1.1. gewonnenen IgG
      10ml des aus 1.1.1. gewonnenen Antiserums wurden mit 10ml gesättigtem Ammoniumsulfat (pH 7,3) gemischt, inkubiert bei 20°C für 30min. und dann bei 4.000g für 10min. abzentifugiert. Der Überstand wurde verworfen, der Niederschlag in 20ml 50% gesättigtem Ammoniumsulfat resuspendiert (gewaschen) und wieder abzentrifugiert. Der Niederschlag wurde in 10ml PBS aufgenommen und die gesamten Reinigungsschritte wurden wiederholt. Danach wurde das gereinigte Protein gegen PBS bei 4°C über 48Std. mit 2 maligen Wechseln des PBS dialysiert. Durch Röhrchengelelektrophorese im SDS-PAGE (6,5% Gel) und anschließender Färbung mit Amidoschwarz, konnte der Reinheitsgrad der gewonnenen Immunglobulinfraktion festgestellt werden. Aus 10ml Vollserum wurde 40mg Immunglobulin (überwiegend IgG) erhalten, durch Einengung mit Hilfe einer Amicon Ultrafiltrationszelle (XM50 Membran) wurde die Proteinkonzentration auf 20mg/ml gebracht.
   1.2.1. Isolierung des humanem IgM
      Als IgM Quelle wurde ein Plasmafiltrat (Plasmapherese) eines Patienten mit IgM Paraproteinämie (Morbus Waldenström) benutzt. Dieses Material bestand bereits zu über 80% aus IgM. Eine einmalige Fällung mit 50% Ammoniumsulfat (wie in 1.1.2. beschrieben) wurde vorgenommen. Die Proteine wurden dann durch Gelchromatographie mit Hilfe einer S.300 Sephacryl Säule (Pharmacia) weitergereinigt (Laufpuffer PBS, Säule 3x65cm, Durchflußrate 13ml/Std., 5ml Fraktionen, Auftragsmenge: 30mg). Die IgM haltigen Fraktionen wurden durch SDS-Page in einem 6% Gel nachgewiesen, gepoolt und eingeengt wie oben (1.1.2.) beschrieben. Nach Dialyse gegen PBS wies das IgM Präparat eine Proteinkonzentration von 10mg/ml auf, es wurde sterilfiltriert und bei 4°C aufbewahrt.
1.3. Kovalentes Verbinden von IgG und IgM mittels Dimethylsuberimidat
   Die Herstellung des Heterodimers, bestehend aus dem gemäss Beispiel 1.1.2. gereinigten, spezifisch gegen Borrelia burgdorferi aus Kaninchen reaktiven IgG und dem gemäss Beispiel 1.2.1. gereinigten humanem IgM erfolgte im wesentlichen nach der von GONDOLF; K. B. et al. Virchows Archiv B Cell Pathol. 60, 353 - 363, 1991 beschriebenen Methode, die sich auf die Originalbeschreibung von DUTTON, A. et al., Biochem. Biophys. Res. Comm. 23, 730 - 739, 1966 und von LUDWIG, M. L. und HUNTER, M. J., Methods of Enzymology Vol. XI(Hirs, C.H.W., Ed), Academic Press, 595 - 604, 1967, bezieht. Es wurden 20mg des Kaninchen IgG und 5mg des menschlichen IgM in einem Gesamtvolumen von 1,5ml in PBS mit 1N NaOH auf pH 10 eingestellt. Anschließend wurden 5mg Dimethylsuberimidat (Pierce, Holland) bei Raumtemperatur unter Rühren portionsweise, innerhalb von 30 Minuten, hinzugegeben. Der pH Wert wurde mit 0,1N NaOH konstant bei pH10 gehalten, nach weiteren 30min. bei Raumtemperatur wurde die Reaktionslösung in der Kälte bei 4-8 °C gegen PBS für 48 Stunden (2xwechseln) dialysiert. Nach der Dialyse betrug der pH Wert 7.2. Die Reaktionslösung wurde danach bei 9.000g für 3min. zentrifugiert. Eine weitere Reinigung über Gelchromatographie wurde ausprobiert, dieser Schritt erwies sich als nicht vorteilhaft und wurde danach nicht mehr angewendet.

### Beispiel 2: Funktionsnachweis des IgG:lgM chimären Antikörpers

### 2.1 Testsysteme und Durchführung der Teste.

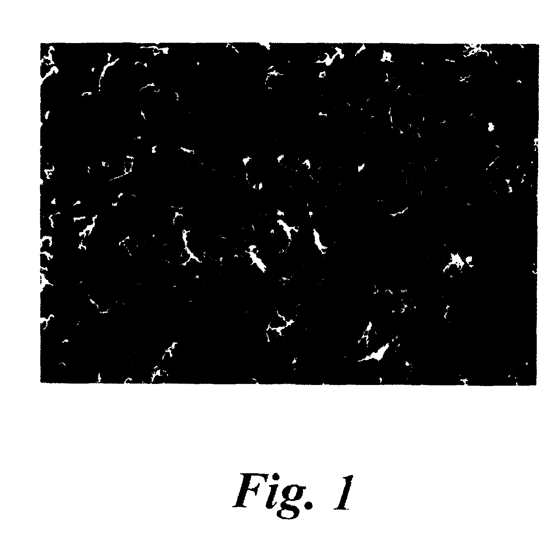

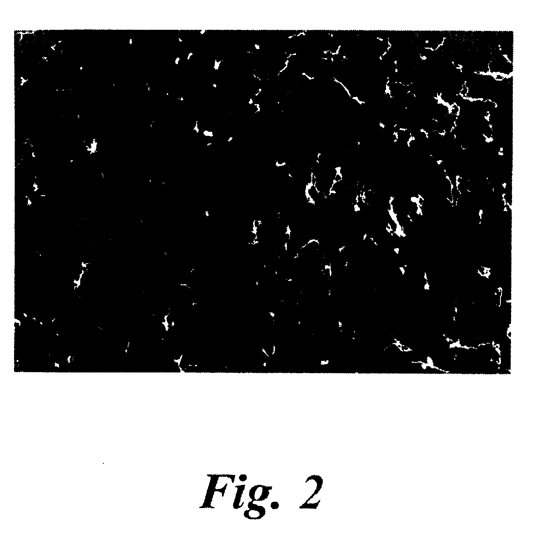

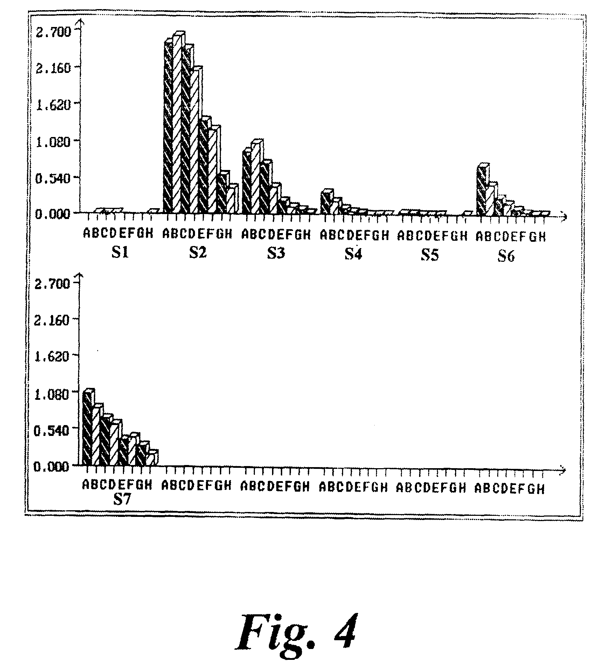

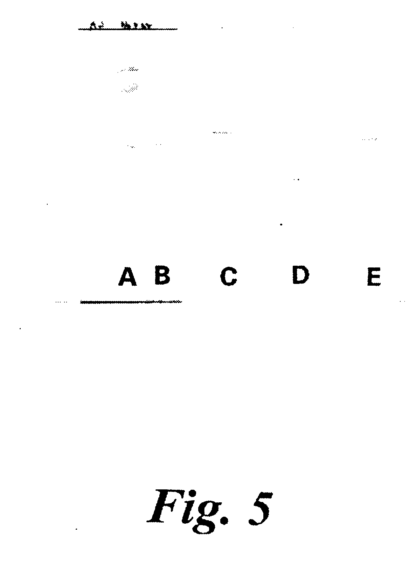

2.1.1. Indirekte Immunfluoreszenz:
   1) *Borrelia burgdorferi* B31 (ATCC 35270) wurde angezüchtet und aufbereitet wie in 1.1.1. beschrieben. Die in 0,5ml PBS resuspendierten Borrelien wurden 1:40 bis 1:160 in PBS weiter verdünnt (die im Test optimale Verdünnung mußte ermittelt werden).
   2) Objektträger (OT's) (4mm Felder, 12 Felder/OT) wurden mit 10µl/Feld der Bakterien Suspension beschichtet, bei 37°C für 45min. staubfrei getrocknet und anschliessend in Aceton bei -20°C für 10min, fixiert.
   3) Vom Testserum wurde eine geometrische Verdünnungsreihe (ab 1:20) in PBS (enthält 5% normales Kaninchenserum) angesetzt und 10µl jeder Verdünnung pro Feld (Spot) aufgetragen und 30min. bei 37°C inkubiert. 4) Danach wurden die OT's 10min. in PBS-Tween (0,1 % Tween 20) und 10min, in PBS gewaschen.
   5) 10µl Anti-Human IgM FITC (1:80 verdünnt, Fa. Behring) pro Spot wurde aufgetropft und 30min. bei 37°C inkubiert.
   6) Danach wurden die OT's 2xl0min. in PBS gewaschen.
   7) Nach dem Abtrocknen der OT's wurde 5µl Eindeckmedium (PBS/Glycerol, 9:1) pro Spot aufgetragen und einen Deckglas aufgelegt. Die Auswertung erfolgte mit einem Zeiss Axiomat Fluoreszenz Mikroskop bei 400 facher Vergrößerung (Filterkombination 10, BP450-490, FT510, BP515-565).
2.1.2. ELISA:
   1) Für die Beschichtung der Mikrotiterplatten wurden 100µl Antigen-Verdünnung pro Vertiefung eingegeben und 12Std. bei 40°C in einer feuchten Kammer absorbieren gelassen. Als Antigenpräparat wurde ein Ultrasonikat von *Borrelia burgdorferi* auf 1µg/ml eingestellt. Als Kopplungspuffer wurde Na-Carbonat, 0,05M, pH9.6 verwendet.
   2) Die Platten wurden 5 Mal in PBS-Tween 20 (2%) gewaschen.
   3) Danach wurde 100µl der in PBS verdünnten Seren pro Vertiefung eingegeben und 1Std. bei 37°C inkubiert.
   4) Anschließend wurde 5 Mal in PBS-Tween gewaschen.
   5) Es wurden 50µl Peroxidase markierte Anti-Human IgM-Ak zugegeben (Verdünnung 1:3.000) und 1Std. bei 37°C inkubiert.
   6) Es wurde wieder 5 Mal in PBS-Tween waschen.
   7) 100µl Substrat pro Vertiefung wurden eingegeben und 10min. bei 37°C inkubiert. Substratlösung: Na₂HPO₄, 3.61g, Citronensäure-1-Hydrat 2.07g, *o*-Phenylendiamin 0,04g in 100ml H₂O, direkt vor Gebrauch 2µl H₂O₂/10ml dazugegeben. Die Reaktion wurde mit 50µl/Vertiefung 2M H₂SO₄ gestoppt und die Extinktion bei 492 nm in einem ELISA Reader gemessen (Fa. SLT, 74564 Crailsheim, Deutschland).
2.1.3. Westernblot:
   1) Das Antigen wurde in der Sodium-Dodecyl-Sulfat Polyacrylamidgel-Elektrophorese (SDS-PAGE) aufgetrennt (12,6% Gel, Antigen - *Borrelia burgdorferi* Ultrasonicat, 2µg/Spur).
   2) Das Gel wurde herausgenommen und die aufgetrennte Proteine auf Immobilon (PVDF) Membran transferiert (Elektrotransfer 20mA, 12Std. ), danach wurde die Membran in 6mm Streifen geschnitten.
   3) Die Testseren wurden in 1ml PBS + 10% Milch verdünnt und bei 4°C über Nacht mit der Streifen inkubiert (Verdünnung z.B.1:100).
   4) Die Streifen wurden 3 Mal für 5min. in PBS-Tween 20 (0,1%) gewaschen.
   5) Danach wurde 1ml zweiter Peroxidase markierte Anti-Human-lgM Antikörper in Arbeitsverdünnung (1:3.000) pro Streifen zugegeben.
   6) Es wurde wie in 4) gewaschen
   7) Anschließend wurden die Streifen in Diaminobenzidin (DAB) (Sigma, Deisenhofen, Deutschland) entwickelt (20mg DAB in 30ml PBS + 24µl H₂O₂).
2.2 Ergebnisse
   2.2.1. Indirekte Immunfluoreszenz: Das IgG/IgM *Borrelia burgdorferi* (Bb) Hybrid (IgG = Anti-Bb B31) zeigte eine starke Fluoreszenz der Borrelien (Fig. 1), der Endtiter war 1:800 (das Ausgangsserum von Kaninchen hatte einen Titer von 1:1280). Ein IgM Antikörper positives Patienten Serum zeigte einen Titer von 1:128 (Fig.2). Ein negatives Resultat (negatives Patienten Serum, bzw. lgG/lgM Hybrid ohne Borrelienspezifität) zeigte ab einer Verdünnug von 1:20 keine spezifische Fluoreszenz.
   2.2.2. ELISA: Die Extinktionswerte einer Mikrotiterplatte sind in Fig.4 gezeigt. Ausgangsverdünnung bei allen Proben war 1:100. Auswahl der negativen und positiven Seren anhand der Immunfluoreszenz (IF) Ergebnisse.
      Die nachstehende Tabelle zeigt die erhaltenen Einzelwerte der Mikrotiterplatte (Verdünnungen A bis H und Spalten 1 bis 7 wie in der Legende zu Fig.4 beschrieben). 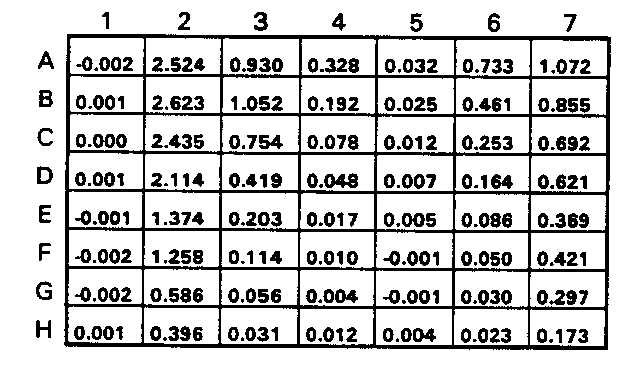
   2.2.3. Westernblot: Die Proben wurden alle bei einer Verdünnung von 1:100 bis 1:500 getestet. Das IgG/IgM Bb Hybrid war stark positiv, ein positives Patientenserum (IgM pos. in IF) war ebenfalls positiv. Dahingegen zeigen sowohl das negative IgG/IgM Kontrollhybrid als auch ein negatives Patientenserum keinerlei Reaktionen (Fig.5).
   2.2.4. Stabilitätsuntersuchungen: verglichen wurden IgG/lgM Bb Hybrid mit einem positiven humanem IgM Serum in IF, sowohl bei Lagerung bei 4°C als auch in gefrorenem Zustand. Nach 30 Wochen bei 4°C war der Titer des Hybrids gleich geblieben, wobei der Titer des Patientenserums stark zurückgegangen war (Grenzwertig). Nach 30 Wochen bei -20°C zeigte das Hybrid ebenfalls keine Abnahme des Titers, der Titer des Patientenserums nahm um etwa 2 Titerstufen ab.

## Patentansprüche

1. Chimärer Antikörper gemäß der allgemeinen Formel
Y₁-Z-Y₂,
worin Y₁ ein erstes Immunglobulin der allgemeinen Formel IgX ist und nicht-humanem Ursprungs ist und worin X für G, M, A, E oder D und deren Isotypen steht, Z eine direkte oder indirekte kovalente Bindung bedeutet und Y₂ ein zweites Immunglobulin der allgemeinen Formel IgX und humanem Ursprungs ist mit der oben definierten Bedeutung von X.

2. Chimärer Antikörper nach Anspruch 1, **dadurch gekennzeichnet**, daß Y₁ ein hochaffines IgX mit bekannter Spezifität und Y₂ ein IgX mit unbekannter und von Y₁ verschiedener Spezifität ist.

3. Chimärer Antikörper nach Ansprüchen 1 und 2, **dadurch gekennzeichnet**, daß Z eine indirekte kovalente Bindung bedeutet.

4. Chimärer Antikörper nach Anspruch 3, **dadurch gekennzeichnet**, daß Z die Bedeutung eines Linker- bzw. Verbindungsmoleküls hat, mit dem Y₁ und Y₂ miteinander kovalent verbunden ist.

5. Chimärer Antikörper nach Anspruch 4, **dadurch gekennzeichnet,** daß das Linker- bzw. Verbindungsmolekül ein homobifunktionelles Linker- bzw. Verbindungsmolekül ist.

6. Chimärer Antikörper nach Anspruch 5, **dadurch gekennzeichnet**, daß das homobifunktionelle Verbindungsmolekül aminspezifisch ist.

7. Chimärer Antikörper nach Anspruch 6, **dadurch gekennzeichnet**, daß das homobifunktionelle Verbindungsmolekül ein Imidoester ist.

8. Chimärer Antikörper nach Anspruch 7, **dadurch gekennzeichnet**, daß der Imidoester ein Bisimidat ist.

9. Chimärer Antikörper nach Anspruch 8, **dadurch gekennzeichnet**, daß das Bisimidat die Verbindung Dimethylsuberimidat ist.

10. Chimärer Antikörper nach Ansprüchen 1 und 2, **dadurch gekennzeichnet,** daß Z eine direkte kovalente Bindung ist.

11. Chimärer Antikörper nach Anspruch 10, **dadurch gekennzeichnet**, daß die direkte kovalente Bindung eine S-S - Bindung ist.

12. Chimärer Antikörper nach einem der Ansprüche 1 bis 11, **dadurch gekennzeichnet**, daß Y₂ ein IgM bedeutet.

13. Chimärer Antikörper nach einem der Ansprüche 1 bis 12, **dadurch gekennzeichnet**, daß Y₁ ein IgG bedeutet.

14. Chimärer Antikörper nach Anspruch 13, **dadurch gekennzeichnet**, daß Y₁ ein IgG mit einer Spezifität gegen Spirochaeten ist.

15. Chimärer Antikörper nach Anspruch 14, **dadurch gekennzeichnet**, daß Y₁ ein IgG mit einer Spezifität gegen Borrelien ist.

16. Chimärer Antikörper nach Ansprüchen 1 bis 15, **dadurch gekennzeichnet,** daß Y₁ und/oder Y₂ ein Antikörperfragment ist.

17. Chimärer Antikörper nach Ansprüchen 1 bis 16, **dadurch gekennzeichnet**, daß er eine Markersubstanz enthält.

18. Chimärer Antikörper nach Anspruch 17, **dadurch gekennzeichnet**, daß die Markersubstanz ein Enzym, ein Fluoreszenzmarker oder ein radioaktives Isotop ist.

19. Verwendung eines chimären Antikörpers nach einem der Ansprüche 1 bis 18 in immundiagnostischen Verfahren und Immunoassays.

20. Verwendung eines chimären Antikörpers nach Anspruch 19 als Kontrollmittel zur Überprüfung eines immundiagnostischen Verfahrens oder Immunoassays.

21. Verfahren zur Herstellung eines chimären Antikörpers nach Ansprüchen 1 bis 18, **gekennzeichnet durch** die Schritte
a) Immunisierung eines Tieres mit einer gewünschten immunogen wirkenden Substanz,
b) Reinigung des aus Schritt a) gewonnenen Immunglobulins IgX,
c) Isolierung eines Immunglobulins IgX aus dem Blutserum eines Menschen,
d) Reinigung des aus Schritt c) gewonnenen Immunglobulins IgX,
e) Bildung eines chimären Antikörpers gemäß der allgemeinen Formel Y₁-Z-Y₂ mit der in Ansprüchen 1 oder 2 gegebenen Definition für Y₁, Z und Y₂ durch direkte oder indirekte Bindung zwischen den aus Schritten b) und d) gewonnenen Immunglobulinen

22. Verfahren nach Anspruch 21, **dadurch gekennzeichnet,** daß der aus Schritt e) gebildete chimäre Antikörper zusätzlich mit einer Markersubstanz konjugiert wird.

23. Verfahren nach Ansprüchen 21 und 22, **dadurch gekennzeichnet**, daß die Bildung des über Schritt e) gewonnenen chimären Antikörpers durch Reaktion mit einem Linker- bzw. Verbindungsmolekül, ausgewählt aus den Gruppen der heterobifunktionellen und homobifunktionellen Verbindungsmolekülen, erfolgt.

24. Verfahren nach Anspruch 23, **dadurch gekennzeichnet,** daß die Verbindungsmoleküle gemäß Ansprüchen 6 bis 9 definiert sind.

25. Verfahren nach Anspruch 21, **dadurch gekennzeichnet,** daß die Bildung des über Schritt e) gewonnenen chimären Antikörpers durch Ausbildung einer S-S - Bindung erfolgt.

26. Verfahren zur Überprüfung eines immundiagnostischen Verfahrens oder eines Immunoassays, **dadurch gekennzeichnet**, daß ein chimärer Antikörper gemäß Ansprüchen 1 bis 18 oder eine Kombination davon verwendet wird.

27. Verfahren nach Anspruch 26, **gekennzeichnet durch** die Schritte
a) Inkontaktbringen eines chimärer Antikörpers nach Ansprüchen 1 bis 18 mit einem gewünschten Immunogen oder Antigen,
b) Unterwerfung des aus a) gebildeten Komplexes eines serologisch-immunologischen Verfahrens oder Testsystems,
c) Auswertung auf Basis des in Schritt b) gewählten Verfahrens oder Testsystems

28. Diagnostisches Mittel mindestens einen chimären Antikörper nach einem der Anspüche 1 bis 18 enthaltend.

29. Kit enthaltend einen chimären Antikörper nach einem der Ansprüche 1 bis 18 und mindestens einen mit einem bestimmten Antigen beschichteten Objektträger.

30. Kit nach Anspruch 29, **dadurch gekennzeichnet**, daß er zusätzlich ein IgM spezifisches Antihumanglobulin und einen Puffer enthält.

31. Kit nach Anspruch 30, **dadurch gekennzeichnet**, daß das IgM spezifische Antihumanglobulin fluoreszenzmarkiert ist.

## Claims

1. Chimeric antibody according to general formula
Y₁-Z-Y₂,
wherein Y₁ denotes a first immunoglobulin of general formula IgX and is of non-human origin and wherein X denotes G, M, A, E or D and the isotypes thereof, Z denotes a direct or indirect covalent bond and Y₂ is a second immunoglobulin of general formula IgX and of human origin with X defined as hereinbefore.

2. Chimeric antibody according to claim 1, characterised in that Y₁ is a high affinity IgX with known specificity and Y₂ is an IgX with unknown specificity different from Y₁.

3. Chimeric antibody according to claims 1 and 2, characterised in that Z is an indirect covalent bond.

4. Chimeric antibody according to claim 3, characterised in that Z denotes a linker or connecting molecule by which Y₁ and Y2 are covalently bound to one another.

5. Chimeric antibody according to claim 4, characterised in that the linker or connecting molecule is a homobifunctional linker or connecting molecule.

6. Chimeric antibody according to claim 5, characterised in that the homobifunctional connecting molecule is amine-specific.

7. Chimeric antibody according to claim 6, characterised in that the homobifunctional molecule is an imidoester.

8. Chimeric antibody according to claim 7, characterised in that the imidoester is a bisimidate.

9. Chimeric antibody according to claim 8, characterised in that the bisimidate is the compound dimethylsuberimidate.

10. Chimeric antibody according to claims land 2, characterised in that Z is a direct covalent bound.

11. Chimeric antibody according to claim 10, characterised in that the direct covalent bound is a S-S bond.

12. Chimeric antibody according to claims 1 to 11, characterised in that Y₂ denotes an IgM.

13. Chimeric antibody according to one of claims 1to 12, characterised in that Y₁ denotes an IgG.

14. Chimeric antibody according to claim 13, characterised in that Y₁ is an IgG with specificity against spirochaetes.

15. Chimeric antibody according to claim 14, characterised in that Y₁ is an IgG with specificity against Borrelia.

16. Chimeric antibody according to claims 1 to 15, characterised in that Y₁ and/or Y₂ is an antibody fragment.

17. Chimeric antibody according to claims 1 to 16, characterised in that it contains a marker substance.

18. Chimeric antibody according to claim 17, characterised in that the marker substance is an enzyme, a fluorescence marker or a radioactive isotope.

19. Use of a chimeric antibody according to one of claims 1 to 18 in immunodiagnostic processes and immunoassays.

20. Use of a chimeric antibody according to claim 19 as a control agent for checking an immunodiagnostic process or immunoassay.

21. Process for producing a chimeric antibody according to claims 1 to 18, characterised by the steps of
a) immunising an animal with a desired immunogenically acting substance,
b) purifying the immunoglobulin IgX obtained from step a),
c) isolating an immunogobulin IgX from the blood serum of a human,
d) purifying the immunoglobulin IgX obtained from step c),
e) forming a chimeric antibody according to general formula Y₁-Z-Y₂ with Y₁, Z and Y₂ defined as in claim 1 or 2 by direct or indirect binding between the immunoglobulins obtained from steps b) and d).

22. Process according to claim 21, characterised in that the chimeric antibody formed from step e) is additionally conjugated with a marker substance.

23. Process according to claims 21 and 22, characterised in that the chimeric antibody obtained using step e) is formed by reaction with a linker or connecting molecule selected from the groups of the heterobifunctional and homobifunctional connecting molecules.

24. Process according to claim 23, characterised in that the connecting molecules are defined as in claims 6 to 9.

25. Process according to claim 21, characterised in that the chimeric antibody obtained using step e) is formed by the production of an S-S bond.

26. Processs for checking an immunodiagnostic method or an immunoassay, characterised in that a chimeric antibody according to claims 1 to 18 or a combination thereof is used.

27. Process according to claim 26, characterised by the steps of
a) contacting a chimeric antibody according to claims 1 to 18 with a desired immunogen or antigen,
b) subjecting the complex formed in a) to a serological/immunological process or test system,
c) evaluating on the basis of the process or test system selected in step b).

28. Diagnostic agent containing at least one chimeric antibody according to one of claims 1 to 18.

29. Kit containing a chimeric antibody according to one of claims 1 to 18 and at least one slide coated with a specific antigen.

30. Kit according to claim 29, characterised in that it additionally contains an IgM specific antihuman globulin and a buffer.

31. Kit according to claim 30, characterised in that the IgM specific antihuman globulin is fluorescent-labelled.

## Revendications

1. Anticorps chimère selon la formule générale
Y₁-Z-Y₂
où Y₁ est une première immunoglobuline de formule générale Igx et est d'origine non humaine et où X représente G, M, A, E ou D ou leurs isotypes, Z représente une liaison covalente directe ou indirecte et Y₂ est une deuxième immunoglobuline de formule générale IgX et est d'origine humaine avec la signification de X définie ci-dessus.

2. Anticorps chimère selon la revendication 1 caractérisé en ce que Y₁ est une IgX à haute affinité de spécificité connue et Y₂ est une IgX de spécificité inconnue et différente de Y₁.

3. Anticorps chimère selon les revendications 1 et 2 caractérisé en ce que Z représente une liaison covalente indirecte.

4. Anticorps chimère selon la revendication 3 caractérisé en ce que Z a la signification d'une molécule lieur ou de liaison avec laquelle Y₁ et Y₂ sont liés l'un à l'autre de manière covalente.

5. Anticorps chimère selon la revendication 4 caractérisé en ce que la molécule lieur ou de liaison est une molécule lieur ou de liaison homobifonctionnelle.

6. Anticorps chimère selon la revendication 5 caractérisé en ce que la molécule de liaison homobifonctionnelle est aminospécifique.

7. Anticorps chimère selon la revendication 6 caractérisé en ce que la molécule de liaison homobifonctionnelle est un imidoester.

8. Anticorps chimère selon la revendication 7 caractérisé en ce que l'imidoester est un bisimidate.

9. Anticorps chimère selon la revendication 8 caractérisé en ce que le bisimidate est le composé subérimidate de diméthyle.

10. Anticorps chimère selon les revendications 1 et 2 caractérisé en ce que Z est une liaison covalente directe.

11. Anticorps chimère selon la revendication 10 caractérisé en ce que la liaison covalente directe est une liaison S-S.

12. Anticorps chimère selon l'une des revendications 1 à 11 caractérisé en ce que Y₂ représente une IgM.

13. Anticorps chimère selon l'une des revendications 1 à 12 caractérisé en ce que Y₁ représente une IgG.

14. Anticorps chimère selon la revendication 13 caractérisé en ce que Y₁ est une IgG ayant une spécificité contre les spirochètes.

15. Anticorps chimère selon la revendication 14 caractérisé en ce que Y₁ est une IgG ayant une spécificité contre les borellia.

16. Anticorps chimère selon les revendications 1 à 15 caractérisé en ce que Y₁ et/ou Y₂ est/sont un fragment d'anticorps.

17. Anticorps chimère selon les revendications 1 à 16 caractérisé en ce qu'il contient une substance marqueur.

18. Anticorps chimère selon la revendication 17 caractérisé en ce que la substance marqueur est une enzyme, un marqueur fluorescent ou un isotope radioactif.

19. Utilisation d'un anticorps chimère selon l'une des revendications 1 à 18 dans des procédés immunodiagnostiques et des immunodosages.

20. Utilisation d'un anticorps chimère selon la revendication 19 comme agent de contrôle pour tester un procédé immunodiagnostique où des immunodosages.

21. Procédé de préparation d'un anticorps chimère selon les revendications 1 à 18 caractérisé par les étapes de
a) immunisation d'un animal avec une substance à effet immunogène voulue,
b) purification de l'immunoglobuline IgX obtenue à partir de l'étape a),
c) isolement d'une immunoglobuline IgX à partir du sérum sanguin d'un humain,
d) purification de l'immunoglobuline IgX obtenue à partir de l'étape c),
e) formation d'un anticorps chimère selon la formule générale Y₁-Z-Y₂ avec la définition donnée dans la revendication 1 ou 2 pour Y₁, Z et Y₂ par liaison directe ou indirecte entre les immunoglobulines obtenues à partir des étapes b) et d).

22. Procédé selon la revendication 21 caractérisé en ce que l'anticorps chimère formé à partir de l'étape e) est conjugué en outre avec une substance marqueur.

23. Procédé selon les revendications 21 et 22 caractérisé en ce que la formation de l'anticorps chimère obtenu par le biais de l'étape e) a lieu par réaction avec une molécule lieur ou de liaison choisie dans les groupes des molécules de liaison hétérobifonctionnelles et des molécules de liaison homobifonctionnelles.

24. Procédé selon la revendication 23 caractérisé en ce que les molécules de liaison sont définies selon les revendications 6 à 9.

25. Procédé selon la revendication 21 caractérisé en ce que la formation de l'anticorps chimère obtenu par le biais de l'étape e) a lieu par formation d'une liaison S-S.

26. Procédé pour tester un procédé immunodiagnostique ou un immunoessai caractérisé en ce que l'on utilise un anticorps chimère selon les revendications 1 à 18 ou une combinaison de ceux-ci.

27. Procédé selon la revendication 26 caractérisé par les étapes de
a) mise en contact d'un anticorps chimère selon les revendications 1 à 18 avec un immunogène ou antigène voulu,
b) soumission du complexe formé à partir de a) à un procédé ou système de test sérologique-immunologique,
c) évaluation sur la base du procédé ou système de test choisi dans l'étape b).

28. Agent diagnostique contenant au moins un anticorps chimère selon l'une des revendications 1 à 18.

29. Kit contenant un anticorps chimère selon l'une des revendications 1 à 18 et au moins un porte-objet recouvert d'un antigène déterminé.

30. Kit selon la revendication 29 caractérisé en ce qu'il contient en outre un antiglobuline humaine spécifique d'IgM et un tampon.

31. Kit selon la revendication 30 caractérisé en ce que l'antiglobuline humaine spécifique d'IgM est marqué par fluorescence.
